(19) Europäisches Patentamt
European Patent Office
Office européen des brevets

(11) EP 3 244 194 A1

(12) **EUROPEAN PATENT APPLICATION**
published in accordance with Art. 153(4) EPC

(43) Date of publication:
**15.11.2017 Bulletin 2017/46**

(21) Application number: **16764709.8**

(22) Date of filing: **03.03.2016**

(51) Int Cl.:
***G01N 21/359*** *(2014.01)* ***G01N 21/3554*** *(2014.01)*
***G01N 33/02*** *(2006.01)*

(86) International application number:
**PCT/JP2016/056558**

(87) International publication number:
**WO 2016/147879 (22.09.2016 Gazette 2016/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **18.03.2015 JP 2015054399**

(71) Applicant: **Mayekawa Mfg. Co., Ltd.**
**Koto-ku**
**Tokyo 135-8482 (JP)**

(72) Inventors:
- **IMAMURA, Hikaru**
  **Tokyo 135-8482 (JP)**
- **KONO, Shinji**
  **Tokyo 135-8482 (JP)**

(74) Representative: **Regimbeau**
**20, rue de Chazelles**
**75847 Paris Cedex 17 (FR)**

# (54) DEVICE FOR DETECTING PHASE TRANSITION CHANGES AND METHOD FOR DETECTING PHASE TRANSITION CHANGES IN MOISTURE-CONTAINING FOOD SAMPLE

(57) An apparatus for detecting a phase transition of a food sample includes: a support base for supporting a food sample which contains moisture and which is able to undergo phase transition between a solid and a liquid; a light penetration unit for permitting near-infrared light containing a light having a wavelength of absorption peak of water and a light having a wavelength of absorption peak of ice to penetrate into the food sample supported by the support base; a spectroscopic unit for dispersing the near-infrared light which is from the light penetration unit and which has transmitted and reflected in the food sample, and measuring a light intensity over a time; an absorbance-and-ice-weight-ratio calculation unit for calculating, on the basis of a spectroscopic data of the near-infrared light obtained by the spectroscopic unit, an absorbance of water and an absorbance of ice in the food sample and calculating an ice weight ratio with respect to a frozen moisture; an absorbance differential calculation unit for calculating a differential of the absorbance of water and a differential of the absorbance of ice which are calculated by the absorbance-and-ice-weight-ratio calculation unit; a calibration curve preparation unit for preparing a calibration curve on the basis of the differential of the absorbance calculated by the absorbance differential calculation unit and the ice weight ratio with respect to the frozen moisture calculated by the absorbance-and-ice-weight-ratio calculation unit; and an ice-weight-ratio estimation unit for estimating, on the basis of the calibration curve prepared by the calibration curve preparation unit, an unknown ice weight ratio from a differential of the absorbance calculated from the absorbance calculated by the absorbance-and-ice-weight-ratio calculation unit.

FIG. 5

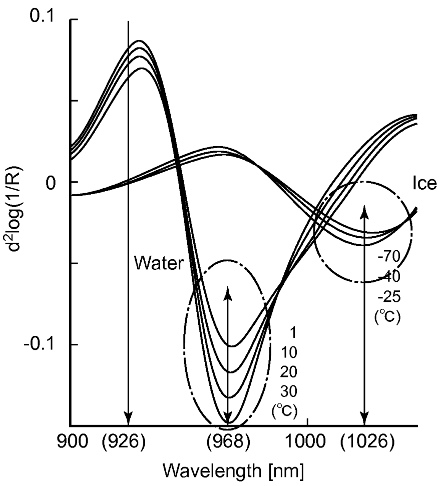


EP 3 244 194 A1

## Description

TECHNICAL FIELD

The present invention relates to an apparatus and a method for detecting phase transition change of a food sample containing moisture, which enables non-destructive detection of phase transition change from a solid to a liquid in a food sample such as frozen meat.

BACKGROUND

In food processing plants, frozen and carried food material needs to be thawed as a preprocessing. Energy required for thawing varies depending on the composition or the shape of the food, and if applied energy is inadequate, a frozen portion may remain in the food, which may have a bad influence on the following process. If applied energy is excess, the quality may be deteriorated. Thus, in order to appropriately control the energy to be applied for thawing and in order to thaw frozen food without deterioration in the quality, it is important to accurately monitor and determine the thawing state of the food material.

At the present day, in order to grasp a thawing state, it is typical to insert a temperature sensor into a food and check whether the food is completely thawed from the central temperature. Further, there is a method for grasp a thawing state without destruction in which a temperature of a material to be thawed is measured by means of infrared light irradiation using e.g. a radiation thermometer (see Patent Document 1). The thawing apparatus disclosed in Patent Document 1 includes: a container body part for containing a material to be thawed; a container accommodation part for holding the container body part; a heating part for thawing the material to be thawed in the container body part via the container accommodation part, which heating part is mounted on an outer circumferential surface of the container accommodation part; and a radiation thermometer for measuring a temperature of a bottom surface of the container body part, which bottom surface is exposed via an opening part which opens to a bottom surface of the container accommodation part.

By a typical method including inserting a temperature sensor, the food of which thawing state has been checked cannot be get back to the process line from a hygienic viewpoint. Further, by such a method, inspection of all pieces cannot be performed, and thus it is not possible to grasp the individual differences (variability in thawing). On the other hand, by a method using a radiation thermometer, since it measures only surface temperatures, it is possible to check the inside thawing state. Further, if a food is irradiated with infrared light, the food may be heated, and the accuracy of the temperature detection may be decreased. Furthermore, by these methods including measuring temperatures, although it is possible to determine whether thawing is completed, since a food in a latent heat region does not show a change in temperature, it is not possible to grasp the progress status of thawing.

On the other hand, in the fields of medicine and agriculture, near-infrared spectroscopy is widely used. Infrared spectroscopy enables non-destructive and non-penetrative measurements by its nature, and since it does not heat a sample, it is used for qualitative and quantitative analysis of a composition particularly in the food field (see Patent Document 2). In the near-infrared spectroscopic analysis disclosed in Patent Document 2, a sample of which protein content is already known is irradiated with near-infrared light with a specific range of wavelengths that protein absorbs; the absorbance is obtained; the absorbance is subjected to second differentiation to calculate a second differential absorbance; a regression analysis is performed where the explanatory variable includes the second differential absorbance and the objective variable is the protein content of the sample, to obtain a calibration curve; and a protein content of an unknown sample is estimated from the calibration curve.

Citation List

Patent Literature

Patent Document 1: JP2014-189501A

Patent Document 2: JPH05-60685A

## SUMMARY

### Technical Problem

As described above, by the near-infrared spectroscopic analysis disclosed in Patent Document 2, it is possible to estimate a protein content of an unknown sample. If it becomes possible to employ a near-infrared spectroscopic analysis as to a phase transition change of a frozen sample during thawing of the sample, it becomes possible to perform non-destructive and non-penetrative measurement of a phase transition change of a sample without heating the sample. Thus, a method and an apparatus using a near-infrared spectroscopic analysis whereby a phase transition change of a food sample can be estimated without destruction are desired.

In view of the above circumstances, at least an embodiment of the present invention is to provide an apparatus and a method for detecting a phase transition change of a food sample containing moisture, which enables non-destructive estimation of a phase transition change of the food sample without heating the food sample, on estimation of the phase transition state of the food sample containing moisture.

### Solution to Problem

An apparatus for detecting a phase transition change of a food sample containing moisture according to at least an embodiment of the present invention comprises: a support base for supporting a food sample which contains moisture and which is able to undergo phase transition from a solid to a liquid; a light penetration unit for permitting near-infrared light containing a light having a wavelength of absorption peak of water and a light having a wavelength of absorption peak of ice to penetrate into the food sample supported by the support base; a spectroscopic unit for dispersing the near-infrared light which is from the light penetration unit and which has transmitted and reflected in the food sample, and measuring a light intensity over a time; an absorbance-and-ice- weight-ratio calculation unit for calculating, on the basis of a spectroscopic data of the near-infrared light obtained by the spectroscopic unit, an absorbance of water and an absorbance of ice in the food sample and calculating an ice weight ratio with respect to a frozen moisture; an absorbance differential calculation unit for calculating a differential of the absorbance of water and a differential of the absorbance of ice which are calculated by the absorbance-and-ice-weight-ratio calculation unit; a calibration curve preparation unit for preparing a calibration curve on the basis of the differential of the absorbance calculated by the absorbance differential calculation unit and the ice weight ratio with respect to the frozen moisture calculated by the absorbance-and-ice-weight-ratio calculation unit; and an ice-weight-ratio estimation unit for estimating, on the basis of the calibration curve prepared by the calibration curve preparation unit, an unknown ice weight ratio from a differential of the absorbance calculated from the absorbance calculated by the absorbance-and-ice-weight-ratio calculation unit.

With the above apparatus for detecting a phase transition change of a food sample containing moisture, the absorbance-and-ice-weight-ratio calculation unit calculates, on the basis of a spectroscopic data of the near-infrared light obtained by the spectroscopic unit, an absorbance of water and an absorbance of ice in the food sample, and an ice weight ratio with respect to a frozen moisture, and the absorbance differential calculation unit calculates a differential of the absorbance of water and a differential of the absorbance of ice which are calculated by the absorbance-and-ice-weight-ratio calculation unit, and the calibration curve preparation unit prepares a calibration curve on the basis of the differential of the absorbance calculated by the absorbance differential calculation unit and the ice weight ratio with respect to the frozen moisture calculated by the absorbance-and-ice-weight-ratio calculation unit. Thus, by detecting an absorbance of a food sample and calculating a differential of the absorbance, an ice weight ratio of a frozen moisture can be estimated from the differential by using the calibration curve. Thus, it is possible to estimate a phase transition change of the food sample during thawing without destruction by heating the food sample. Further, since near-infrared light has a nature of being likely to transmit in a material, the food sample may not be heated when irradiated with near-infrared light. Thus, it is possible to provide an apparatus for detecting a phase transition change of a food sample containing moisture, which enables non-destructive estimation of a phase transition change of the food sample without heating, on estimation of the phase transition state of the food sample containing moisture.

In some embodiments, the differential of the absorbance of water and the differential of the absorbance of ice are a second differential of the absorbance of water and a second differential of the absorbance of ice.

With the above configuration, the differential of the absorbance of water and the differential of the absorbance of ice calculated by the absorbance differential calculation unit are a second differential of the absorbance of water and a second differential of the absorbance of ice, whereby it is possible to express a magnitude of degree of change in the absorbance of water and ice and thereby to clarify the absorption peaks of water and ice. Thus, it is possible to more increase the accuracy of the ice weight ratio estimated by the ice-weight-ratio estimation unit.

In some embodiments, the calibration curve preparation unit is configured to perform a regression analysis in which an objective variable is the calculated ice weight ratio with respect to the frozen moisture and explanatory variables include the calculated second differential of the absorbance of water and the calculated second differential of the absorbance of ice to prepare the calibration curve.

With the above configuration, the calibration curve preparation unit performs a regression analysis in which an objective variable is the calculated ice weight ratio with respect to the frozen moisture and explanatory variables include the calculated second differentials of the absorbance of water and the absorbance of ice to prepare the calibration curve. In this case, the absorption peaks of water and ice are clarified by the second differentials of the absorbances, whereby it is possible to make stronger the correlation between the ice weight ratio of the frozen moisture and the second differentials of the absorbances of water and ice. Thus, it is possible to increase the accuracy of the calibration curve prepared through the regression analysis.

In some embodiments, the light penetration unit includes a light source which is able to emit near-infrared light with wavelengths range of from 750 nm to 1500 nm and which is able to emit near-infrared light containing a light having a wavelength of absorption peak of water and a light having a wavelength of absorption peak of ice.

With the above configuration, the light penetration unit includes a light source which is able to emit near-infrared light with wavelengths range of from 750 nm to 1500 nm and which is able to emit near-infrared light containing a light having a wavelength of absorption peak of water and a light having a wavelength of absorption peak of ice. Thus, the near-infrared light to transmit and reflect in the food sample and to be received by the spectroscopic unit is measured within a wide wavelength range including a near-infrared light with a wavelength of the absorption peak of water and a near-infrared light with a wavelength of the absorption peak of ice, whereby it is possible to remove an influence by a noise at a specific wavelength, and thereby to further improve the accuracy of the spectroscopic data of the near-infrared light measured by the spectroscopic unit.

In some embodiments, the food sample containing moisture includes an internal organ, meat, a heated viscous food containing a mixture of the internal organ and the meat, or a gel-state food.

With the above configuration, an internal organ, meat, a heated viscous food containing a mixture of the internal organ and the meat, or a gel-state food contains moisture. The moisture turns to a solid and undergoes phase transition to a liquid with an increase in the temperature. Thus, since an internal organ, meat, a heated viscous food containing a mixture of the internal organ and the meat, or a gel-state food is able to undergo phase transition change from a solid to a liquid, it is possible to detect a phase transition change of an internal organ, meat, a heated viscous food containing a mixture of the internal organ and the meat, or a gel-state food, by the above apparatus for detecting a phase transition change of a food sample.

A method for detecting a phase transition change of a food sample containing moisture according to at least an embodiment of the present invention comprises: applying near-infrared light containing a light having a wavelength of absorption peak of water and a light having a wavelength of absorption peak of ice to a food sample which contains moisture and which is able to undergo phase transition from a solid to a liquid, calculating an absorbance of water and an absorbance of ice in the food sample over a time, and calculating an ice weight ratio with respect to a frozen moisture; preparing a calibration curve on the basis of a differential of the calculated absorbance and the ice weight ratio with respect to the frozen moisture; and estimating, on the basis of the prepared calibration curve, an unknown ice weight ratio from a differential of the calculated absorbance.

By the above method for detecting a phase transition change of a food sample containing moisture, on the basis of the spectroscopic data of the near-infrared light measured by the spectroscopic unit, an absorbance of water and an absorbance of ice in the food, and an ice ratio by weigh in a frozen moisture are calculated over a time, a differentials of the calculated absorbance of water and ice are calculated, and on the basis of the calculated differentials of the absorbance and the calculated ice weight ratio of the frozen moisture, a calibration curve is prepared. Thus, if the differential of the absorbance can be calculated, the ice weight ratio of a frozen moisture can be estimated from the differential by using the calibration curve. Thus, it is possible to estimate a phase transition change of a food sample containing moisture without destruction by heating the food sample. Further, since near-infrared light has a nature of being likely to transmit in a material, the food sample may not be heated when irradiated with near-infrared light. Thus, it is possible to provide a method for detecting a phase transition change of a food sample containing moisture, which enables non-destructive estimation of a phase transition change of the food sample without heating, on estimation of the phase transition state of the food sample containing moisture.

In some embodiments, the method includes calculating a second differential of the calculated absorbance, and performing a regression analysis in which an objective variable is the calculated ice weight ratio with respect to the frozen moisture and explanatory variables include a calculated second differential of the absorbance of water and a calculated second differential of the absorbance of ice to prepare the calibration curve.

By the above method, the calibration curve is prepared by calculating a second differential of the calculated absorbance, and performing a regression analysis in which an objective variable is the calculated ice weight ratio with respect to the frozen moisture and explanatory variables include a calculated second differential of the absorbance of water and a calculated second differential of the absorbance of ice. Since the absorption peaks of water and ice may be clarified by the second differential of the absorbance, the correlation between the ice weight ratio of the frozen moisture and the second differentials of the absorbance of water and ice is strong. Thus, it is possible to increase the accuracy of the calibration curve prepared through the regression analysis.

In some embodiments, the food sample containing moisture includes an internal organ, meat, a heated viscous food containing a mixture of the internal organ and the meat, or a gel-state food.

By the above method, an internal organ, meat, a heated viscous food containing a mixture of the internal organ and the meat, or a gel-state food contains moisture. The moisture turns to a solid and undergoes phase transition to a liquid with an increase in the temperature. Thus, since an internal organ, meat, a heated viscous food containing a mixture of the internal organ and the meat, or a gel-state food is able to undergo phase transition change from a solid to a liquid, it is possible to detect a phase transition change of an internal organ, meat, a heated viscous food containing a mixture of the internal organ and the meat, or a gel-state food, by the above method for detecting a phase transition change of a food sample.

Advantageous Effects

According to at least some embodiments of the present invention, an apparatus and a method for detecting a phase transition change of a food sample containing moisture, which enables non-destructive estimation of a phase transition change of the food sample without heating, on estimation of the phase transition state of the food sample containing moisture, is provided.

BRIEF DESCRIPTION OF DRAWINGS

FIG. 1 is a schematic diagram illustrating a whole construction of an apparatus for detecting a phase transition change of a food sample according to an embodiment of the present invention.
FIG. 2 is a diagram illustrating an internal construction of a support base, where a food sample supported by the support base is irradiated with near-infrared light.
FIG. 3 is an enlarged view of a potion denoted by A in FIG. 2.
FIG. 4 is a block diagram of an apparatus for detecting a phase transition change of a food sample.
FIG. 5 is a graph showing an absorption spectrum of near-infrared light having absorption peaks of water and ice at different wavelengths.
FIG. 6 is a flowchart illustrating a method for detecting a phase transition change of a food sample.
FIG. 7 is a graph showing a shift of an absorption spectrum during thawing of distilled water.

Each of FIG. 8A to FIG. 8C is a graph showing a phase transition change of water and ice during thawing in a case of distilled water as a measurement object. Each of FIG. 8A and FIG. 8B shows a relation between second differential of absorbance and ice weight ratio at an absorption peak of water with respect to near-infrared light, and FIG. 8C shows a relation between second differential of absorbance and ice weight ratio at an absorption peak of ice with respect to near-infrared light.

FIG. 9 is a graph showing a result of a multiple regression analysis in a case of distilled water as a measurement object, in which the objective variable is ice weight ratio and the explanatory variables include second differentials of absorbance.

Each of FIG. 10A to FIG. 10C is a graph showing a phase transition change of water and ice during thawing in a case of a cured fish paste (kamaboko) as the food sample. Each of FIG. 10A and FIG. 10B shows a relation between second differential of absorbance and ice weight ratio at an absorption peak of water with respect to near-infrared light, and FIG. 10C shows a relation between second differential of absorbance and ice weight ratio at an absorption peak of ice with respect to near-infrared light.

FIG. 11 is a graph showing a result of a multiple regression analysis in a case of a cured fish paste as the food sample, in which the objective variable is ice weight ratio and the explanatory variables include second differentials of absorbance.

Each of FIG. 12A to FIG. 12C is a graph showing a phase transition change of water and ice during thawing in a case of a surimi (paste) of sea bream as the food sample. Each of FIG. 12A and FIG. 12B shows a relation between second differential of absorbance and ice weight ratio at an absorption peak of water with respect to near-infrared light, and FIG. 12C shows a relation between second differential of absorbance and ice weight ratio at an absorption peak of ice with respect to near-infrared light.

FIG. 13 is a graph showing a result of a multiple regression analysis in a case of a surimi (paste) of sea bream as the food sample, in which the objective variable is ice weight ratio and the explanatory variables include second differentials of absorbance.

Each of FIG. 14A to FIG. 14C is a graph showing a phase transition change of water and ice during thawing in a case of a fillet of tuna as the food sample. Each of FIG. 14A and FIG. 14B shows a relation between second differential of absorbance and ice weight ratio at an absorption peak of water with respect to near-infrared light, and FIG. 14C shows a relation between second differential of absorbance and ice weight ratio at an absorption peak of ice with respect to near-infrared light.

FIG. 15 is a graph showing a result of a multiple regression analysis in a case of a fillet of tuna as the food sample, in which the objective variable is ice weight ratio and the explanatory variables include second differentials of absorbance.

Each of FIG. 16A to FIG. 16C is a graph showing a phase transition change of water and ice during thawing in a case of a ham of pig as the food sample. Each of FIG 16A and FIG. 16B shows a relation between second differential of absorbance and ice weight ratio at an absorption peak of water with respect to near-infrared light, and FIG. 16C shows a relation between second differential of absorbance and ice weight ratio at an absorption peak of ice with respect to near-infrared light.

FIG. 17 is a graph showing a result of a multiple regression analysis in a case of a ham of pig as the food sample, in which the objective variable is ice weight ratio and the explanatory variables include second differentials of absorbance.

Each of FIG. 18A to FIG. 18C is a graph showing a phase transition change of water and ice during thawing in a case of an agar as the food sample. Each of FIG. 18A and FIG. 18B shows a relation between second differential of absorbance and ice weight ratio at an absorption peak of water with respect to near-infrared light, and FIG. 18C shows a relation between second differential of absorbance and ice weight ratio at an absorption peak of ice with respect to near-infrared light.

FIG. 19 is a graph showing a result of a multiple regression analysis in a case of an agar as the food sample, in which the objective variable is ice weight ratio and the explanatory variables include second differentials of absorbance.

DETAILED DESCRIPTION

Embodiments of the apparatus for detecting a phase transition change of a food sample containing moisture and the method for detecting a phase transition will now be described in detail with reference to FIG. 1 to FIG. 19. In the following description, example embodiments where "a cured fish paste", a "surimi (paste)" of sea bream, a "fillet" of tuna, a "ham of pig" and an "agar" are used as the food samples containing moisture. It is intended, however, that unless particularly specified, materials, shapes, relative positions and the like of components described in the embodiments shall be interpreted as illustrative only and not limitative of the scope of the present invention.

Firstly, before describing the method for detecting a phase transition change of a food sample containing moisture according to embodiments of the present invention, embodiments of the apparatus for detecting a phase transition change of a food sample containing moisture will be described. A phase transition change detection apparatus 1 for a food sample containing moisture includes, as illustrated in FIG. 1, a light penetration device 5, a support base 10, a spectroscopic device 30, and an ice-weight-ratio estimation device 40. The light penetration device 5 is a light source which is able to emit near-infrared light, for example a halogen light source.

The near-infrared light emitted from the light penetration device 5 is guided through an optical fiber cable 6 to a food sample S (see FIG. 2) which is supported by the support base 10. The support base 10 includes an accommodation part 11 which is able to accommodate the food sample S, and a leg part 15 extending downward from a bottom portion of the accommodation part 11 to hold the accommodation part 11 at an upper position. The accommodation part 11 has a frame body 18 formed in a cuboid-like shape and a plurality of synthetic-resin plates 12 (e.g. acrylic plates) fitted around an upper part of the frame body 18 to form a box-like shape. Light toward the accommodation part 11 is blocked by the plates 12, whereby intrusion of visible light and near-infrared light from outside into the accommodation part 11 is prevented. The plate 12 on the front side of the accommodation part 11 is configured to be openable and closable, and a food sample S can thereby be put in and taken out of the accommodation part 11.

As illustrated in FIG. 2, a bottom plate 19 on which a food sample S is to be placed is provided in the accommodation part 11 and above the central portion of a plate 12 which constitutes a bottom surface 12b. In this embodiment, the bottom plate 19 is a porous spongy sheet. In the central portion of the bottom plate 19, a pair of hole portions 19a to permit near-infrared light projected from the optical fiber cable 6 to pass through is disposed with a space therebetween in the width direction. The bottom plate 19 may be any plate on which a food sample S can be placed, and it may be a plate made from a synthetic resin.

One end portion of the optical fiber cable 6 is inserted from a downside of a through-hole portion 12a formed in the plate 12 disposed on the bottom surface portion of the accommodation part 11 and is held in the through-hole portion 12a. As illustrated in FIG. 3, a plug 8 having a diameter larger than the optical fiber cable 6 is joined to the one end portion of the optical fiber cable 6. In the plug 8, a lens 9 (e.g. an aberration-free non-spherical lens) is disposed. The lens 9 is configured to apply near-infrared light projected from the optical fiber cable 6 to the food sample S.

In another through-hole portion 12a of the bottom plate 19, one end portion of another optical fiber cable 7 is inserted. A plug 8 having a diameter larger than the optical fiber cable 6 is joined to the one end portion of the optical fiber cable 7. In the plug 8, a lens 9 (e.g. an aberration-free non-spherical lens) is disposed. The lens 9 is configured to collect near-infrared light having transmitted and reflected in the food sample S into the optical fiber cable 7. Thus, the near-infrared light projected from one end portion of the optical fiber cable 6 transmits and reflects in the food sample S, and is received by the optical fiber cable 7.

To the other end portion of the optical fiber cable 7, a spectroscopic device 30 is connected. As illustrated in FIG. 1, the spectroscopic device 30 is configured to receive, through the optical fiber cable 7, the near-infrared light which was emitted by the light penetration device 5 and which transmitted and reflected in the food sample S, and to separate the received near-infrared light into every wavelength and to measure the light intensity. The spectroscopic data obtained by the spectroscopic device 30 is sent to the ice-weight-ratio estimation device 40.

As illustrated in FIG. 4, the ice-weight-ratio estimation device 40 is to process the spectroscopic data from the spectroscopic device 30, and it may, for example, be a personal computer. The ice-weight-ratio estimation device 40 includes: an absorbance-and-ice-weight-ratio calculation unit 41 configured, during thawing of a frozen food sample S, to calculate, on the basis of the spectroscopic data from the spectroscopic device 30, an absorbance of water and an absorbance of ice contained in the food sample and to calculate, from the absorbances of water and ice, an ice weight ratio with respect to a frozen moisture; an absorbance differential calculation unit 43 configured to perform a second differentiation of the calculated absorbance to calculate a second differential of absorbance; a calibration curve preparation unit 45 configured to perform a multiple regression analysis in which explanatory variables include the calculated second differential of absorbance and the objective variable is the ice weight ratio with respect to the frozen moisture to prepare the calibration curve; and an ice-weight-ratio estimation unit 47 configured to estimate, on the basis of the calibration curve, an unknown ice weight ratio from a second differential of absorbance calculated from the absorbance calculated by the absorbance-and-ice-weight-ratio calculation unit 41.

Here, near-infrared light will be described. Near-infrared light has a nature such that if near-infrared light is applied to water and ice, a light with a different wavelength is absorbed in the case of water and in the case of ice, respectively. The vertical axis of FIG. 5 indicates degree of absorption of near-infrared light, where the degree of absorption increases as the position on the vertical axis becomes closer to the lowermost part. The horizontal axis of FIG. 5 indicates wavelength of near-infrared light. As shown in FIG. 5, in the case of water, near-infrared light is specifically absorbed at a wavelength of about 968 nm, and in the case of ice, near-infrared light is specifically absorbed at a wavelength of about 1026 nm. The phase transition change detection apparatus 1 for a food sample containing moisture according to this embodiment utilizes such a nature of near-infrared light that in the near-infrared absorption spectrum, the absorption peaks of water and ice are at wavelengths which are different from each other.

Now, a method for detecting a phase transition change of a food sample by using the phase transition change detection apparatus 1 for a food sample containing moisture will be described. Before using a food sample S, a phase transition change of distilled water is tested by using the phase transition change detection apparatus 1 for a food sample S. Firstly, a prescribed amount (e.g. 27 g) of distilled water is put into a petri dish (e.g. having a diameter of φ60 mm) which is not shown in the drawing. Then, the petri dish is placed in a constant-temperature reservoir (e.g. at -35°C) which is not shown in the drawing, to freeze the distilled water. After the distilled water is frozen, the distilled water is placed under an ordinary temperature condition to thaw the distilled water.

During thawing, the petri dish containing the distilled water which is frozen (hereinafter referred to as "frozen distilled water") is placed in the accommodation part 11 of the support base 10, near-infrared light emitted from the light penetration device 5 through the optical fiber cable 6 is applied to the frozen distilled water, and near-infrared light which has transmitted and reflected in the frozen distilled water is received by the spectroscopic device 30 through the optical fiber cable 7 over a time, as illustrated in FIG. 1. The spectroscopic device 30 disperses the received near-infrared light and measures the light intensity. Then, the ice-weight-ratio estimation device 40 calculates an absorbance on the basis of the spectroscopic data (wavelength and light intensity) of the near-infrared light obtained by the spectroscopic device 30. Absorption A is calculated from the following formula.

$$A=-\log_{10}(I/I_0)$$

where $I_0$ is intensity of incident light, and I is intensity of transmitted light.

Then, the ice-weight-ratio estimation device 40 calculates a second differential B of absorbance A with respect to wavelength, from the calculated absorbance A. That is, B is calculated from $B=d^2A/d\lambda^2$.

After measurement of the spectroscopic date, the petri dish is taken out from the support base 10, and the weight of ice in the frozen distilled water after removing melted water in the petri dish is measured. Then, the data on ice weight is input into the ice-weight-ratio estimation device 40, and the ice-weight-ratio estimation device 40 calculates ice weight ratio C on the basis of the data on ice weight. Ice weight ratio C is calculated from the following formula. The weight of ice is measured with e.g. a weighing scale by an operator.

$$C=100\times(\text{ice amount [g]})/(\text{total water amount [g]})$$

FIG. 7 is a graph showing a relation between wavelength and second differential of absorbance, where the vertical axis indicates such calculated second differential of absorbance, and the horizontal axis indicates wavelength. From FIG. 7, it can be seen that, regardless of the ice weight ratio, the absorption spectrum for water and ice is shifted. That is, it can be seen that near-infrared light includes different wavelengths at which a peak of the absorption spectrum for water and a peak of the absorption spectrum for ice exist, respectively.

Each of FIG. 8A and FIG. 8B is a graph showing a relation between ice weight ratio and second differential of absorbance at a wavelength of an absorption peak of water, where the vertical axis indicates the calculated second differential of absorbance, and the horizontal axis indicates the calculated ice weight ratio. FIG. 8C is a graph showing a relation between ice weight ratio and second differential of absorbance at a wavelength of an absorption peak of ice, where the vertical axis indicates the calculated second differential of absorbance, and the horizontal axis indicates the calculated ice weight ratio. From these graphs, calibration curves $L_1$, $L_2$ and $L_3$, each of which has high accuracy in relation between ice weight ratio and second differential of absorbance (coefficient of determination $R^2$=0.927, 0.963, 0.835), can be drawn.

FIG. 9 is a result of a multiple regression analysis where explanatory variables include the calculated second differential of absorbance, and the objective variable is ice weight ratio in frozen moisture. From FIG. 9, it can be seen that by using combination of second differentials of absorbance, ice weight ratio estimated from the frozen moisture can be expressed in a high accuracy (coefficient of determination $R^2$=0.966).

Now, by using an actual food sample (a cured fish paste (kamaboko)), an embodiment of a method for detecting a phase transition change of a food sample (a cured fish paste) will be described with reference to FIG. 1, FIG. 2 and FIG. 6. Firstly, as illustrated in FIG. 6, a food sample is prepared. In this embodiment, a commercially available "cured fish paste" cut into a piece having a prescribed size (e.g. width: 47 mm, depth: 35 mm, height: 15 mm) is used as the food sample. The food sample S is frozen in a constant-temperature reservoir (for example, the food sample S is frozen at -30°C). After the food sample S is frozen, the food sample S is thawed under an ordinary temperature condition.

During thawing of the food sample S, as illustrated in FIG. 1 and FIG. 2, the food sample S is placed in the accommodation part 11 of the support base 10, near-infrared light emitted from the light penetration device 5 through the optical fiber cable 6 is applied to the food sample S, and near-infrared light which has transmitted and reflected in the food sample S is received by the spectroscopic device 30 through the optical fiber cable 7. The spectroscopic device 30 disperses the received near-infrared light and measures the light intensity. Then, the absorbance-and-ice-weight-ratio calculation unit 41 of the ice-weight-ratio estimation device 40 calculates, on the basis of the measured spectroscopic data (wavelength and light intensity) of the near-infrared light, an absorbance and an ice weight ratio from calculated absorbances A of water and ice. Then, the absorbance differential calculation unit 43 calculates second differential B of absorbance A with respect to wavelength from the calculated absorbance A. Since the method of calculating absorbance A is described above, the same description thereof will be omitted.

After application of the near-infrared light to the food sample S, the food sample S is taken out from the support base 10, and the food sample is thawed on a continuing basis. Then, after a prescribed length of time from when the food sample S is taken out from the support base 10, the food sample S is again placed in the accommodation part 11 of the support base 10, and spectroscopic data (wavelength and light intensity) is measured through the above-described method. Then, the absorbance-and-ice-weight-ratio calculation unit 41 calculates absorbance A and ice weight ratio, and the absorbance differential calculation unit 43 calculates second differential B of absorbance A. In this manner, until the food sample S is completely thawed, spectroscopic data (wavelength and light intensity) is measured, and absorbance A, second differential B of absorbance A, and ice weight ratio are calculated, over a time.

On the other hand, an ice weight ratio of the food sample S (a cured fish paste(kamaboko)) is measured by a calorimetry method. In a calculation method of ice weight ratio through the calorimetry method, firstly, water and a food sample S (a cured fish paste (kamaboko)) are put in a thermally insulating container (e.g. a Dewar vessel), and are stirred until the temperature is uniformed. In this step, the temperatures of the water and the frozen food sample are measured in an initial stage of stirring and after stirring, respectively. In the calorimetry method, an ice weight ratio is calculated from such a relation that, during stirring, an amount of heat $Q_1$ that the water loses is equal to an amount of heat $Q_2$ that the food sample S gains.

The relation between the amount of heat $Q_1$ that the water loses and the amount of heat $Q_2$ that the food sample S gains is as follows:

$$Q_1=G\times(T_f-T_1)\times c_1+G\times r\times A/100+G\times(T-T_f)\times c_2$$

$$Q_2=G_w\times(T_2-T)\times c_w$$

where

- G is weight (g) of the sample,
- $G_w$ is weight (g) of the water,
- $T_1$ is initial temperature (°C) of the sample,
- $T_2$ is initial temperature (°C) of the water,
- $T_f$ is freezing temperature (°C),
- T is final temperature (°C),
- r is latent heat (J/g•°C) of the sample,
- A is ice weight ratio (%),
- $c_1$ is specific heat (J/g•°C) of the sample (at a temperature of at most the freezing point),
- $c_2$ is specific heat (J/g•°C) of the sample (at a temperature of at least the freezing point), and
- $c_w$ is specific heat (J/g•°C) of water(at a temperature of at least the freezing point).

By solving an equation $Q_1=Q_2$, an ice weight ratio by an actual measurement is calculated.

Each of FIG. 10A to FIG. 10C is a graph showing a relation between ice weight ratio and second differential of absorbance, where the horizontal axis indicates the ice weight ratio as calculated above, and the vertical axis indicates the calculated second differential of absorbance. FIG. 10A and FIG. 10B are graphs in the cases of the absorption peak of water at wavelengths of 937 nm and 967 nm, respectively, and FIG. 10C is a graph in the case of the absorption peak of ice at a wavelength of 1022 nm.

On the basis of the calculated second differential of absorbance and the calculated ice weight ratio, the calibration curve preparation unit 45 performs a multiple regression analysis where explanatory variables include the calculated second differential of absorbance and the objective variable is the ice weight ratio in the frozen moisture. Accordingly, calibration curves $L_4$, $L_5$ and $L_6$, each of which has high accuracy in relation between ice weight ratio and second differential of absorbance (coefficient of determination $R^2$=0.955, 0.955, 0.929) are obtainable. On the basis of these calibration curves $L_4$, $L_5$ and $L_6$, an unknown ice weight ratio can be estimated from a second differential of absorbance by the ice-weight-ratio estimation unit 47.

FIG. 11 is a graph showing a relation between the actually measured ice weight ratio and the calculated ice weight ratio, where the vertical axis indicates the calculated ice weight ratio and the horizontal axis indicates the actually measured ice weight ratio, at the absorption peak of ice. It can be seen that an ice weight ratio calculated from this graph has a strong correlation (coefficient of determination $R^2$=0.957) with the actually measured ice weight ratio.

Each of FIG. 12A, FIG. 12B, FIG. 12C and FIG. 13 shows a result of in a case of "a surimi (paste) of sea bream" as the food sample S. A commercially available surimi (paste) of sea bream was cast into a resin mold to be formed into a piece having prescribed dimensions (e.g. width: 35 mm, depth: 35 mm, height: 24 mm), and the piece was used as a sample of a "surimi (paste) of sea bream". The methods for calculating second differential of absorbance and ice weight ratio, and the method (calorimetry method) for measuring ice weight ratio are the same as in the above-described case of the "cured fish paste (kamaboko)" as the food sample S, and thus the description thereof will be omitted.

FIG. 12A and FIG. 12B are graphs showing a relation between ice weight ratio and second differential of absorbance in the cases of the absorption peak of water at wavelengths of 939 nm and 959 nm, respectively, and FIG. 12C is a graph showing a relation between ice weight ratio and second differential of absorbance in the case of the absorption peak of ice at a wavelength of 1025 nm.

On the basis of the calculated second differential of absorbance and the calculated ice weight ratio, the calibration curve preparation unit 45 performs a multiple regression analysis where explanatory variables include the calculated second differential of absorbance and the objective variable is the ice weight ratio in the frozen moisture. Accordingly, calibration curves $L_7$, $L_8$ and $L_9$, each of which has high accuracy in relation between ice weight ratio and second differential of absorbance (coefficient of determination $R^2$=0.935, 0.946, 0.954) are obtainable. On the basis of these calibration curves $L_7$, $L_8$ and $L_9$, an unknown ice weight ratio in the "surimi (paste) of sea bream" can be estimated from a second differential of absorbance by the ice-weight-ratio estimation unit 47.

FIG. 13 is a graph showing a relation between the actually measured ice weight ratio and the calculated ice weight ratio, where the vertical axis indicates the calculated ice weight ratio and the horizontal axis indicates the actually measured ice weight ratio, at the absorption peak of ice. It can be seen that an ice weight ratio calculated from this graph has a strong correlation (coefficient of determination $R^2$=0.955) with the actually measured ice weight ratio.

Each of FIG. 14A, FIG. 14B, FIG. 14 and FIG. 15 shows a result of in a case of a "fillet of tuna" as the food sample S. A commercially available block of tuna was cut into a fillet having prescribed dimensions (e.g. width: 60 mm, depth: 40 mm, height: 40 mm), and the fillet was used as a sample of a "fillet of tuna". The methods for calculating second differential of absorbance and ice weight ratio, and the method (calorimetry method) for measuring ice weight ratio are the same as in the above-described case of the "cured fish paste (kamaboko)" as the food sample S, and thus the description thereof will be omitted.

FIG. 14A and FIG. 14B are graphs showing a relation between ice weight ratio and second differential of absorbance in the cases of the absorption peak of water at wavelengths of 940 nm and 963 nm, respectively, and FIG. 14C is a graph showing a relation between ice weight ratio and second differential of absorbance in the case of the absorption peak of ice at a wavelength of 1030 nm.

On the basis of the calculated second differential of absorbance and the calculated ice weight ratio, the calibration curve preparation unit 45 performs a multiple regression analysis where explanatory variables include the calculated second differential of absorbance and the objective variable is the ice weight ratio in the frozen moisture. Accordingly, calibration curves $L_{10}$, $L_{11}$ and $L_{12}$, each of which has high accuracy in relation between ice weight ratio and second differential of absorbance (coefficient of determination $R^2$=0.702, 0.960, 0.976) are obtainable. On the basis of these calibration curves $L_{10}$, $L_{11}$ and $L_{12}$, an unknown ice weight ratio in the "fillet of tuna" can be estimated from a second differential of absorbance by the ice-weight-ratio estimation unit 47.

FIG. 15 is a graph showing a relation between the actually measured ice weight ratio and the calculated ice weight ratio, where the vertical axis indicates the calculated ice weight ratio and the horizontal axis indicates the actually measured ice weight ratio, at the absorption peak of ice. It can be seen that an ice weight ratio calculated from this graph has a strong correlation (coefficient of determination $R^2$=0.988) with the actually measured ice weight ratio.

Each of FIG. 16A, FIG. 16B, FIG. 16C and FIG. 17 shows a result of in a case of a "ham of pig" as the food sample S. A commercially available block meat of a ham of a pig was cut into a piece having prescribed dimensions (e.g. width: 50 mm, depth: 40 mm, height: 30 mm), and the piece was used as a sample of a "ham of pig". The methods for calculating second differential of absorbance and ice weight ratio, and the method (calorimetry method) for measuring ice weight ratio are the same as in the above-described case of the "cured fish paste (kamaboko)" as the food sample S, and thus the description thereof will be omitted.

FIG. 16A and FIG. 16B are graphs showing a relation between ice weight ratio and second differential of absorbance in the cases of the absorption peak of water at wavelengths of 939 nm and 963 nm, respectively, and FIG. 16C is a graph showing a relation between ice weight ratio and second differential of absorbance in the case of the absorption peak of ice at a wavelength of 1031 nm.

On the basis of the calculated second differential of absorbance and the calculated ice weight ratio, the calibration curve preparation unit 45 performs a multiple regression analysis where explanatory variables include the calculated second differential of absorbance and the objective variable is the ice weight ratio in the frozen moisture. Accordingly, calibration curves L13, L14 and L15, each of which has high accuracy in relation between ice weight ratio and second differential of absorbance (coefficient of determination $R^2$=0.816, 0.979, 0.953) are obtainable. On the basis of these calibration curves L13, L14 and L15, an unknown ice weight ratio in the "ham of pig" can be estimated from a second differential of absorbance by the ice-weight-ratio estimation unit 47.

FIG. 17 is a graph showing a relation between the actually measured ice weight ratio and the calculated ice weight ratio, where the vertical axis indicates the calculated ice weight ratio and the horizontal axis indicates the actually measured ice weight ratio, at the absorption peak of ice. It can be seen that an ice weight ratio calculated from this graph has a strong correlation (coefficient of determination $R^2$=0.985) with the actually measured ice weight ratio.

Each of FIG. 18A, FIG. 18B, FIG. 18C and FIG. 19 shows a result of in a case of an "agar" as the food sample S. A commercially available ager in the form of powder was made into a gel-state agar, and the gel-state agar is cut into a piece having prescribed dimensions (e.g. width: 35 mm, depth: 35 mm, height: 25 mm), and the piece was used as a sample of an "agar". The methods for calculating second differential of absorbance and ice weight ratio, and the method (calorimetry method) for measuring ice weight ratio are the same as in the above-described case of the "cured fish paste (kamaboko)" as the food sample S, and thus the description thereof will be omitted.

FIG. 18A and FIG. 18B are graphs showing a relation between ice weight ratio and second differential of absorbance in the cases of the absorption peak of water at wavelengths of 939 nm and 963 nm, respectively, and FIG. 18C is a graph showing a relation between ice weight ratio and second differential of absorbance in the case of the absorption peak of ice at a wavelength of 1033 nm.

On the basis of the calculated second differential of absorbance and the calculated ice weight ratio, the calibration curve preparation unit 45 performs a multiple regression analysis where explanatory variables include the calculated second differential of absorbance and the objective variable is the ice weight ratio in the frozen moisture. Accordingly, calibration curves L16, L17 and L18, each of which has high accuracy in relation between ice weight ratio and second differential of absorbance (coefficient of determination $R^2$=0.933, 0.966, 0.876) are obtainable. On the basis of these calibration curves L16, L17 and L18, an unknown ice weight ratio in the "agar" can be estimated from a second differential of absorbance by the ice-weight-ratio estimation unit 47.

FIG. 19 is a graph showing a relation between the actually measured ice weight ratio and the calculated ice weight ratio, where the vertical axis indicates the calculated ice weight ratio and the horizontal axis indicates the actually measured ice weight ratio, at the absorption peak of ice. It can be seen that an ice weight ratio calculated from this graph has a strong correlation (coefficient of determination $R^2$=0.967) with the actually measured ice weight ratio.

In the above-described embodiments, an ice weight ratio in the frozen moisture was calculated with a high accuracy from a second differential of absorbance by means of a regression analysis. The ice weight ratio may be calculated in the following manner. When performing a regression analysis, one or more of second differentials of absorbance at wavelengths of about 940 nm, 960 nm and 1020 nm are employed as the explanatory variables to estimate the ice weight ratio. A result of a regression analysis employing a varied number of explanatory variables is shown below. The values in the table are coefficients of determination $R^2$.

| Sample \ Explanatory variable(s) | A Water(940nm) | B Water(960nm) | C Ice (1,020nm) | A,B | B,C | C,A | A,B,C |
|---|---|---|---|---|---|---|---|
| Distilled water | 0.927 | 0.963 | 0.835 | 0.965 | 0.965 | 0.965 | 0.966 |
| Kamaboko | 0.955 | 0.955 | 0.929 | 0.955 | 0.957 | 0.954 | 0.957 |
| Surimi | 0.935 | 0.946 | 0.954 | 0.955 | 0.954 | 0.949 | 0.955 |
| Tuna | 0.702 | 0.960 | 0.976 | 0.975 | 0.982 | 0.984 | 0.988 |
| Ham of pig | 0.816 | 0.979 | 0.953 | 0.979 | 0.982 | 0.984 | 0.985 |
| Agar | 0.933 | 0.966 | 0.876 | 0.967 | 0.967 | 0.964 | 0.967 |

Alternatively, instead of the differential processing, an ice weight ratio can also be calculated with a high accuracy by performing a regression analysis using a value obtained by MSC processing or SNV processing of data on absorbance.

In the above-described embodiments, a halogen light source was used as the light penetration device 5. However, as the light penetration device 5, any light source can be employed by which near-infrared light with wavelengths range of from 750 nm to 1500 nm is obtainable and which emits near-infrared light with wavelengths of about 900 nm to 1100 nm including the wavelength of the absorption peaks of water and ice, and for example, a xenon light source may be used. As the light penetration device 5, a plurality of light sources which emits light with wavelengths of the absorption peaks of water and by which a spectroscopic data, from which a second differential is calculated, is obtainable. In this case, near-infrared light applied to the food sample S includes only a near-infrared light with a wavelength of the absorption peak of water and a near-infrared light with a wavelength of the absorption peak of ice of about 900 to 1100 nm. Accordingly, the near-infrared light having transmitted and reflected in the food sample S and received by the spectroscopic device 30 also includes only a near-infrared light with a wavelength of the absorption peak of water and a near-infrared light with a wavelength of the absorption peak of ice. Thus, it is possible to eliminate an influence by a near-infrared light with another wavelength, and thereby to further improve the accuracy of the spectroscopic data of near-infrared light measured by the spectroscopic device 30.

In the above embodiments, a "cured fish paste (kamaboko)" was described as an example of a heated viscous food. However, instead of a cured fish paste (kamaboko), as heated viscous food, a fish sausage (chikuwa), a minced and steamed fish (hanpen), a ball in Oden, a tube-formed flour paste cake (chikuwabu), a cured fish paste having a swirling pattern (naruto), a minced fish (tsumire) or the like may also be used. In the above embodiments, a "surimi (paste) of sea bream", a "fillet of tuna" and a "ham of pig" were described as examples of meat; however, the meat may, for example, be fish meat of bonito, mackerel or the like, or animal meat of cows, fowl or the like. Further, instead of meat, an internal organ of animals such as cows, pigs or fowl may be used. In the above embodiments, an "agar" was described as an example of a gel-state food; however, as the gel-state food, a gelatin, a bean curd (tohu), a konjac or the like may also be used.

Embodiments of the present invention are described in detail above, but the present invention is not limited thereto, and various modifications may be made without departing from the scope of the invention.

Reference Signs List

1 Phase transition change detection apparatus for food sample containing moisture
5 Light penetration device (Light penetration means)

6,7 Optical fiber cable
10 Support base
11 Accommodation part
12 Plate
12a Through-hole portion
12b Bottom surface
15 Leg part
18 Frame body
19 Bottom plate
19a Hole portion
30 Spectroscopic device (Spectroscopic means)
40 Ice-weight-ratio estimation device
41 Absorbance-and-ice-weight-ratio calculation unit (Absorbance-and-ice-weight-ratio calculation means)
43 Absorbance differential calculation unit (Absorbance differential calculation means)
45 Calibration curve preparation unit (Calibration curve preparation means)
47 Ice-weight-ratio estimation unit (Ice-weight-ratio estimation means)
$L_1$-$L_{18}$ Calibration curve
S Food sample

## Claims

**1.** An apparatus for detecting a phase transition change of a food sample containing moisture, the apparatus comprising:

a support base for supporting a food sample which contains moisture and which is able to undergo phase transition from a solid to a liquid;
a light penetration unit for permitting near-infrared light containing a light having a wavelength of absorption peak of water and a light having a wavelength of absorption peak of ice to penetrate into the food sample supported by the support base;
a spectroscopic unit for dispersing the near-infrared light which is from the light penetration unit and which has transmitted and reflected in the food sample, and measuring a light intensity over a time;
an absorbance-and-ice-weight-ratio calculation unit for calculating, on the basis of a spectroscopic data of the near-infrared light obtained by the spectroscopic unit, an absorbance of water and an absorbance of ice in the food sample and calculating an ice weight ratio with respect to a frozen moisture;
an absorbance differential calculation unit for calculating a differential of the absorbance of water and a differential of the absorbance of ice which are calculated by the absorbance-and-ice-weight-ratio calculation unit;
a calibration curve preparation unit for preparing a calibration curve on the basis of the differential of the absorbance calculated by the absorbance differential calculation unit and the ice weight ratio with respect to the frozen moisture calculated by the absorbance-and-ice-weight-ratio calculation unit; and
an ice-weight-ratio estimation unit for estimating, on the basis of the calibration curve prepared by the calibration curve preparation unit, an unknown ice weight ratio from a differential of the absorbance calculated from the absorbance calculated by the absorbance-and-ice-weight-ratio calculation unit.

**2.** The apparatus for detecting a phase transition change of a food sample containing moisture according to claim 1, wherein the differential of the absorbance of water and the differential of the absorbance of ice calculated by the absorbance differential calculation unit are a second differential of the absorbance of water and a second differential of the absorbance of ice.

**3.** The apparatus for detecting a phase transition change of a food sample containing moisture according to claim 2, wherein the calibration curve preparation unit is configured to perform a regression analysis in which an objective variable is the calculated ice ratio by weight with respect to the frozen moisture and explanatory variables include the calculated second differential of the absorbance of water and the calculated second differential of the absorbance of ice to prepare the calibration curve.

**4.** The apparatus for detecting a phase transition change of a food sample containing moisture according to claim 1, wherein the light penetration unit includes a light source which is able to emit near-infrared light with wavelengths range of from 750 nm to 1500 nm and which is able to emit near-infrared light containing a light having a wavelength of absorption peak of water and a light having a wavelength of absorption peak of ice.

**5.** The apparatus for detecting a phase transition change of a food sample containing moisture according to any one of claims 1 to 4,
wherein the food sample containing moisture includes an internal organ, meat, a heated viscous food containing a mixture of the internal organ and the meat, or a gel-state food.

**6.** A method for detecting a phase transition change of a food sample containing moisture, the method comprising:

applying near-infrared light containing a light having a wavelength of absorption peak of water and a light having a wavelength of absorption peak of ice to a food sample which contains moisture and which is able to undergo phase transition from a solid to a liquid, calculating an absorbance of water and an absorbance of ice in the food sample over a time, and calculating an ice ratio by weight with respect to a frozen moisture from the calculated absorbance of water and the calculated absorbance of ice;
preparing a calibration curve on the basis of a differential of the calculated absorbance and the ice ratio by weight with respect to the frozen moisture; and
estimating, on the basis of the prepared calibration curve, an unknown ice ratio by weight from a differential of the calculated absorbance.

**7.** The method for detecting a phase transition change of a food sample containing moisture according to claim 6, the method comprising:

calculating a second differential of the calculated absorbance, and performing a regression analysis in which an objective variable is the calculated ice ratio by weight with respect to the frozen moisture and explanatory variables include a calculated second differential of the absorbance of water and a calculated second differential of the absorbance of ice to prepare the calibration curve.

**8.** The method for detecting a phase transition change of a food sample containing moisture according to claim 6 or 7, wherein the food sample containing moisture includes an internal organ, meat, a viscous food containing a mixture of the internal organ and the meat, or a gel-state food.

FIG. 1

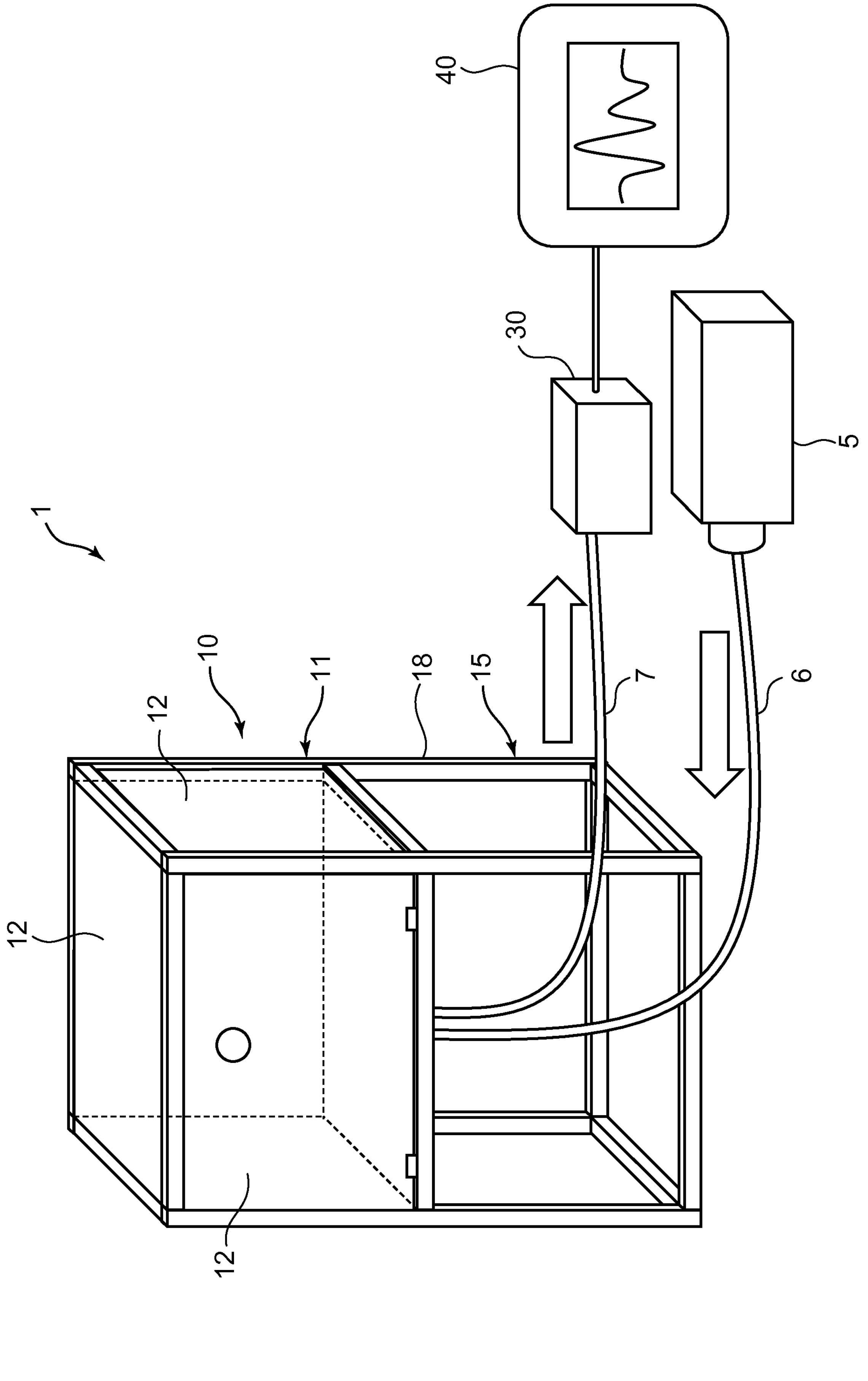

FIG. 2

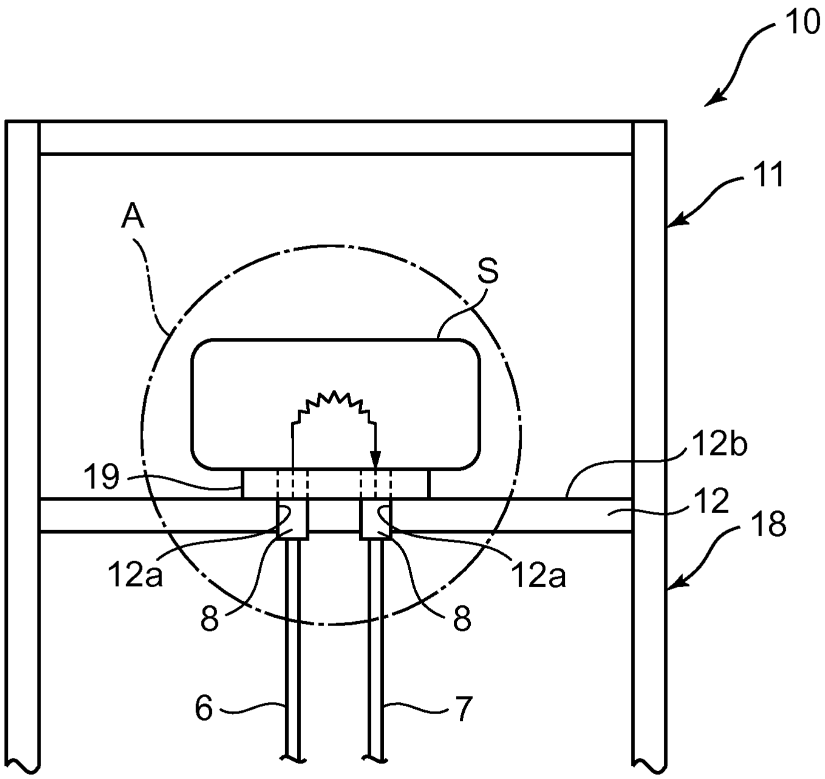
10
11
A
S
12b
19
12
18
12a
12a
8
8
6
7

FIG. 3

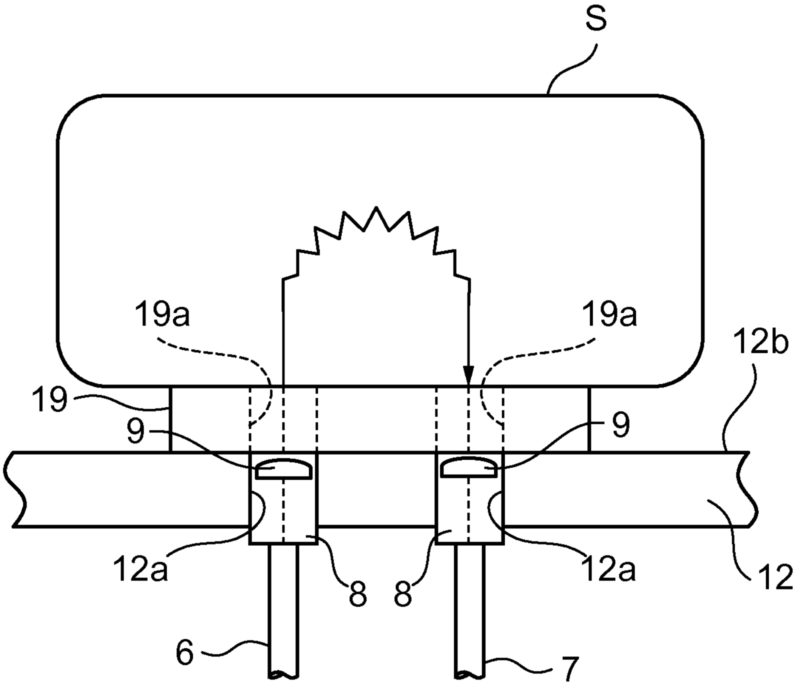
S
19a
19a
12b
19
9
9
12a
8
8
12a
12
6
7

FIG. 4

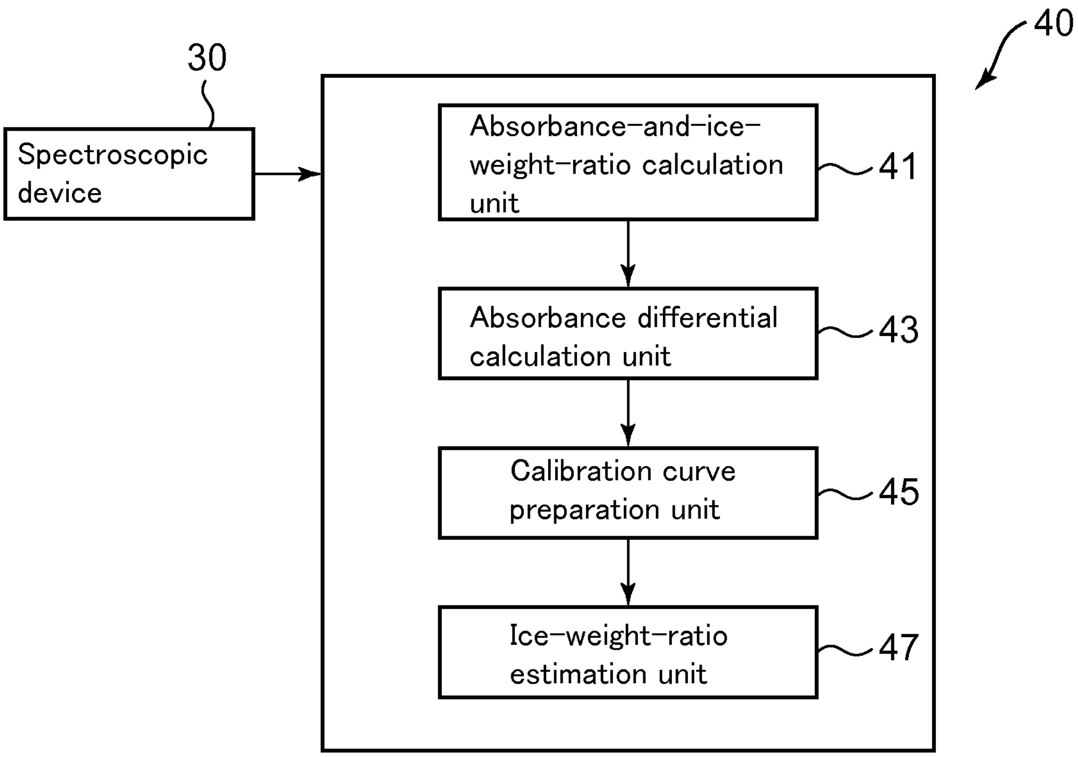
40
30
Spectroscopic device
Absorbance-and-ice-weight-ratio calculation unit
41
Absorbance differential calculation unit
43
Calibration curve preparation unit
45
Ice-weight-ratio estimation unit
47

FIG. 5

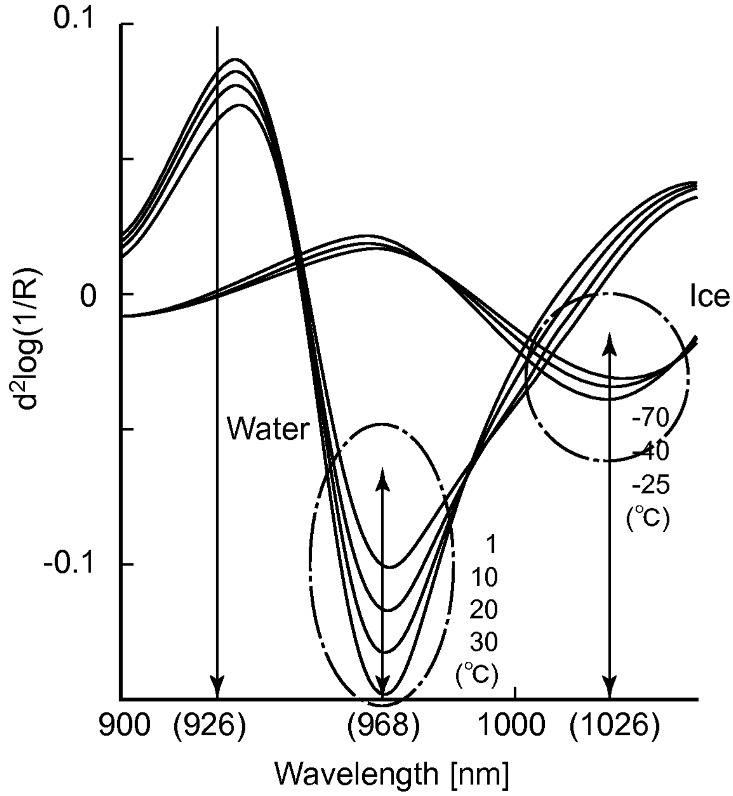

0.1
0
-0.1
d²log(1/R)
Water
Ice
1
10
20
30
(℃)
-70
-40
-25
(℃)
900 (926) (968) 1000 (1026)
Wavelength [nm]


FIG. 6

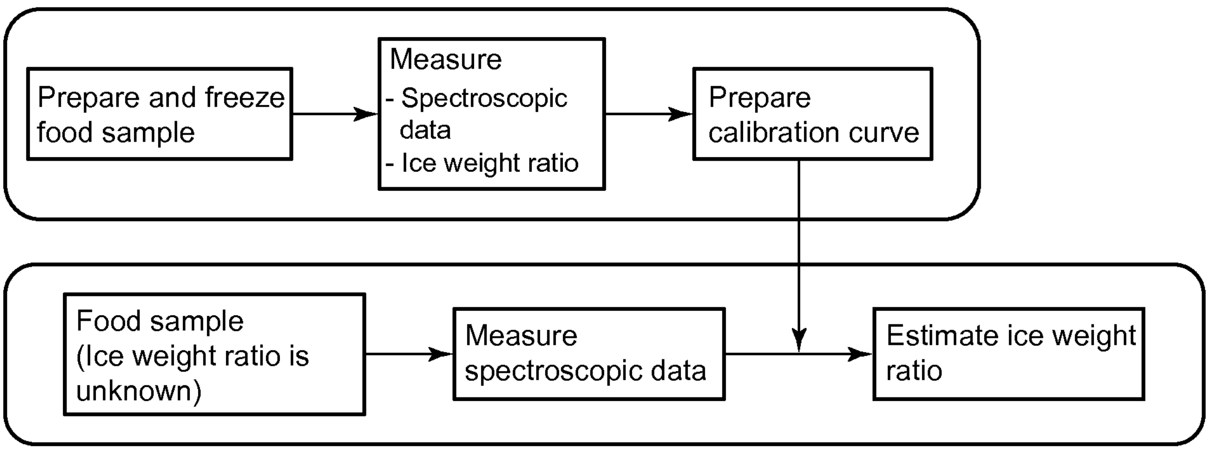

Prepare and freeze food sample
Measure
- Spectroscopic data
- Ice weight ratio
Prepare calibration curve
Food sample (Ice weight ratio is unknown)
Measure spectroscopic data
Estimate ice weight ratio

FIG. 7

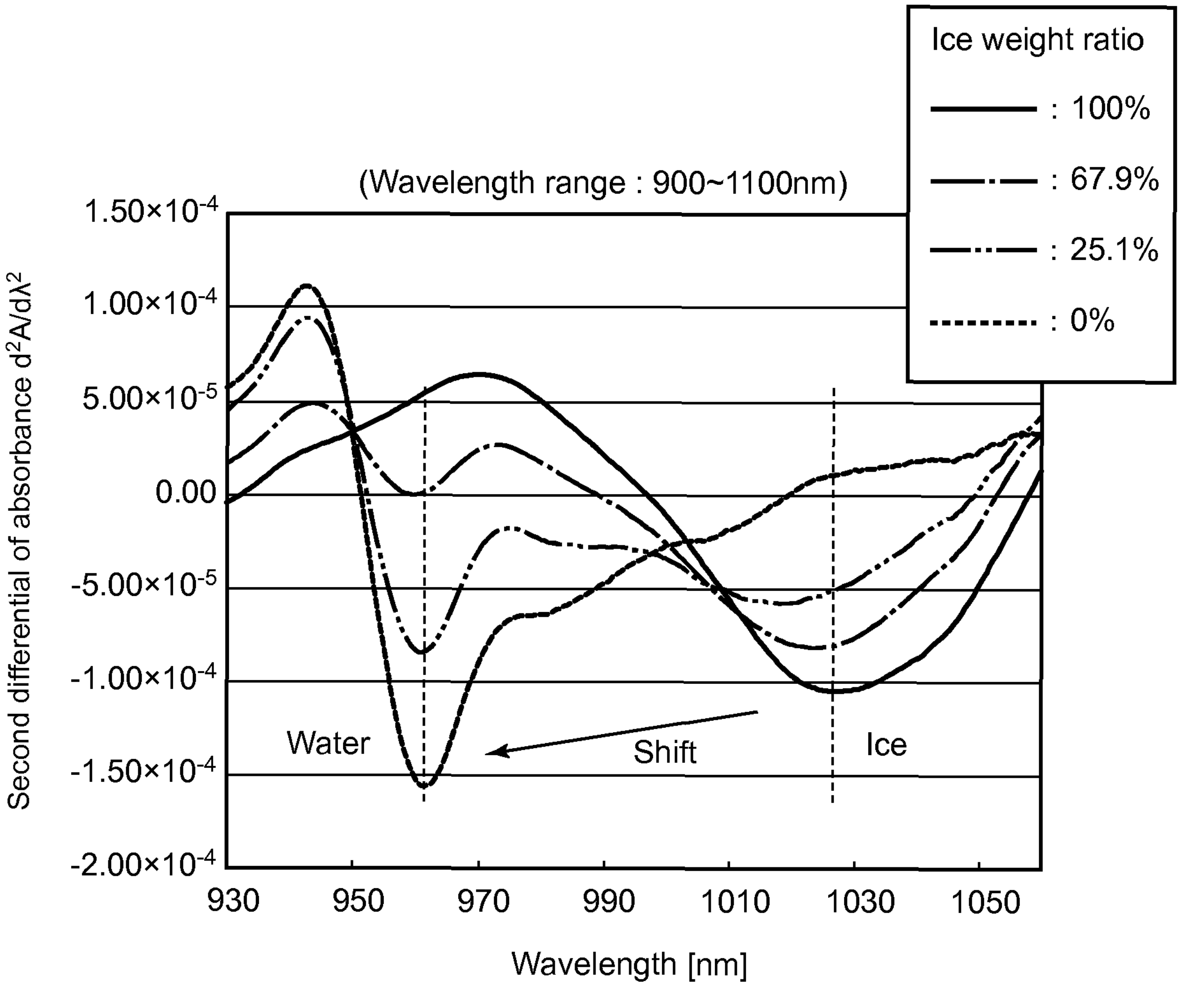
(Wavelength range : 900~1100nm)
Ice weight ratio
: 100%
: 67.9%
: 25.1%
: 0%
1.50×10-4
1.00×10-4
5.00×10-5
0.00
-5.00×10-5
-1.00×10-4
-1.50×10-4
-2.00×10-4
Second differential of absorbance d2A/dλ2
Water
Shift
Ice
930
950
970
990
1010
1030
1050
Wavelength [nm]

FIG. 8A

[Distilled Water]

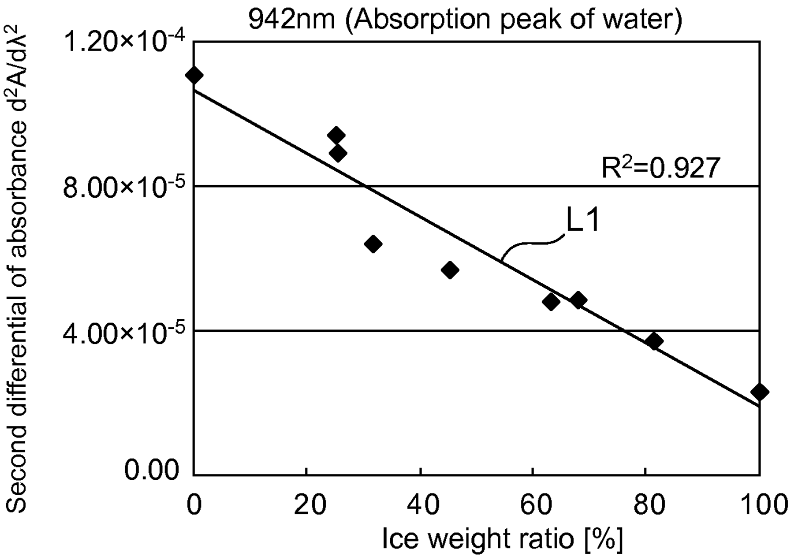


FIG. 8B

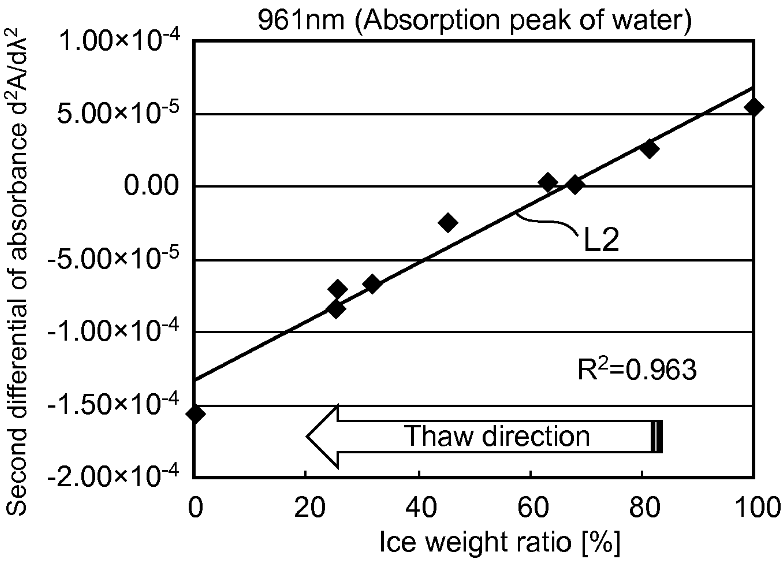


FIG. 8C

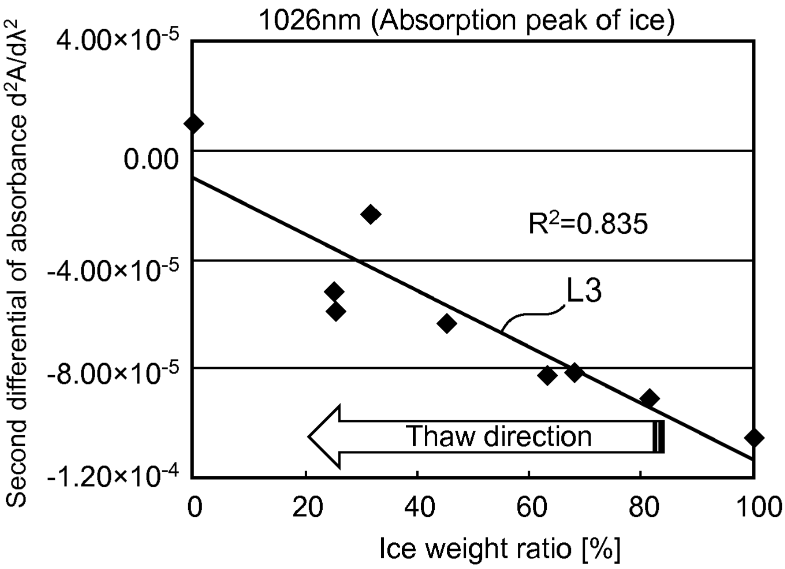

FIG. 9

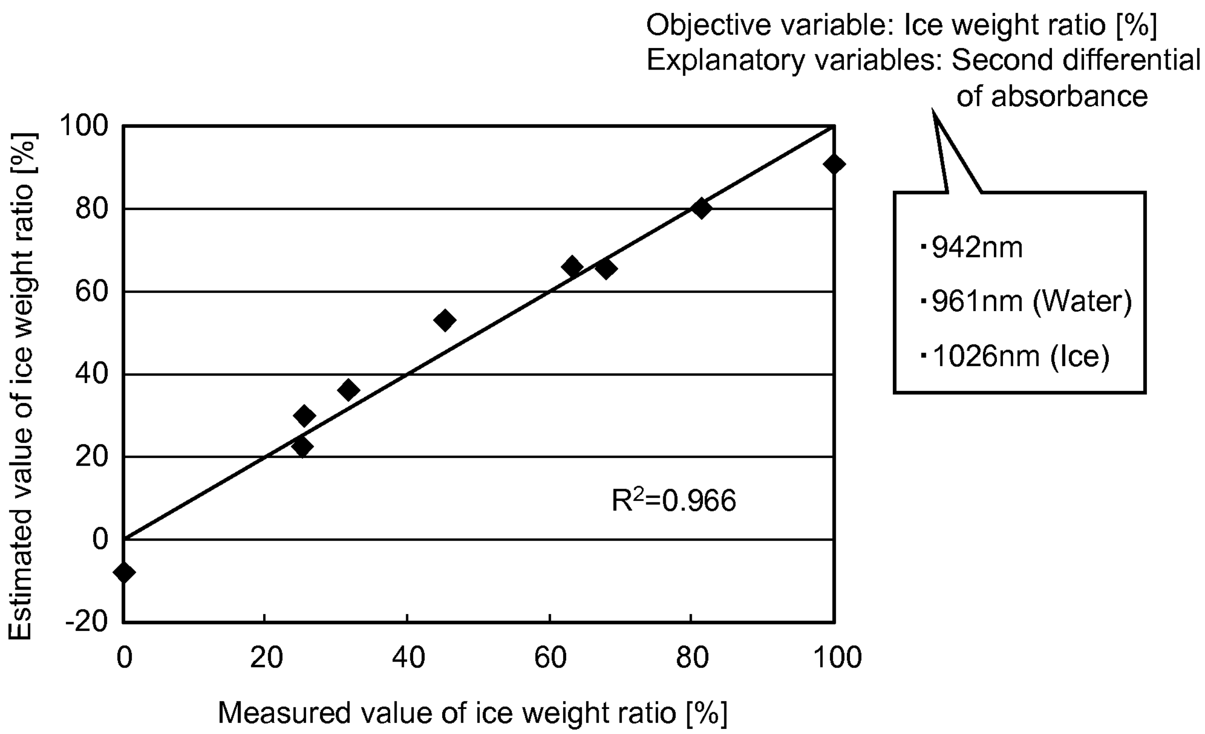
Objective variable: Ice weight ratio [%]
Explanatory variables: Second differential of absorbance
・942nm
・961nm (Water)
・1026nm (Ice)
100
80
60
40
20
0
-20
Estimated value of ice weight ratio [%]
0
20
40
60
80
100
Measured value of ice weight ratio [%]
R2=0.966

FIG. 10A

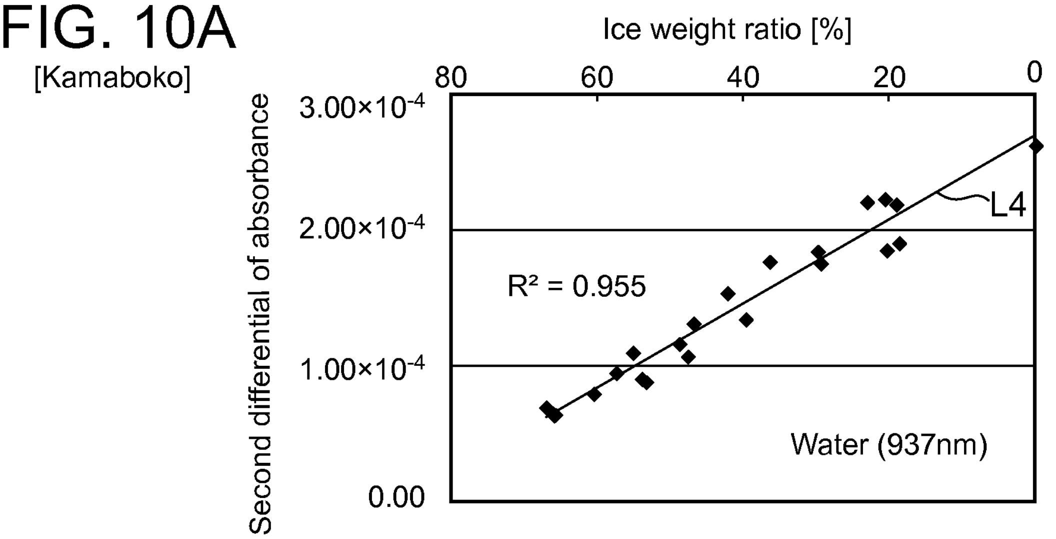


FIG. 10B

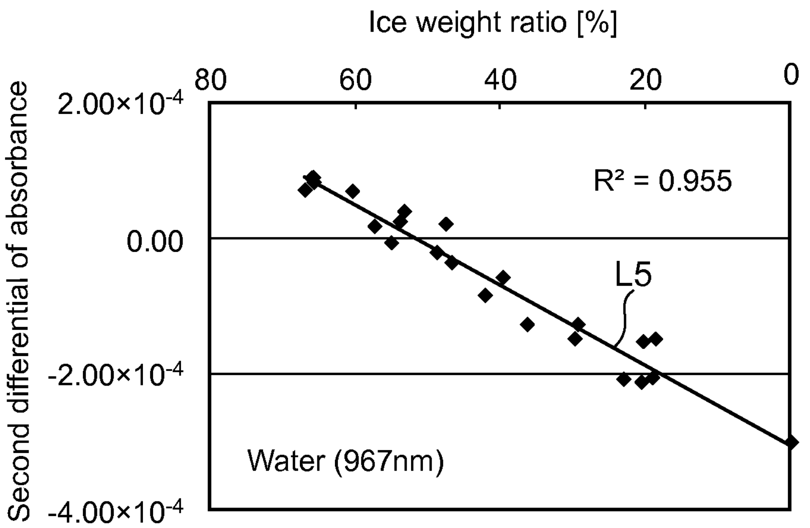


FIG. 10C

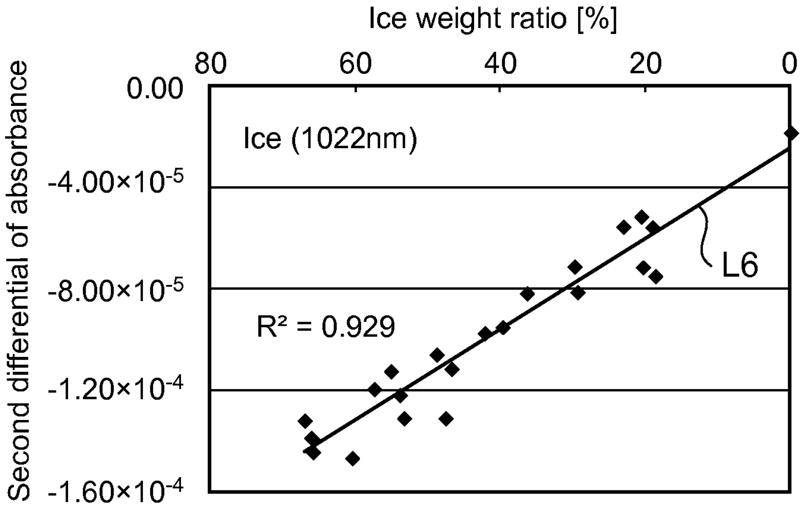

FIG. 11

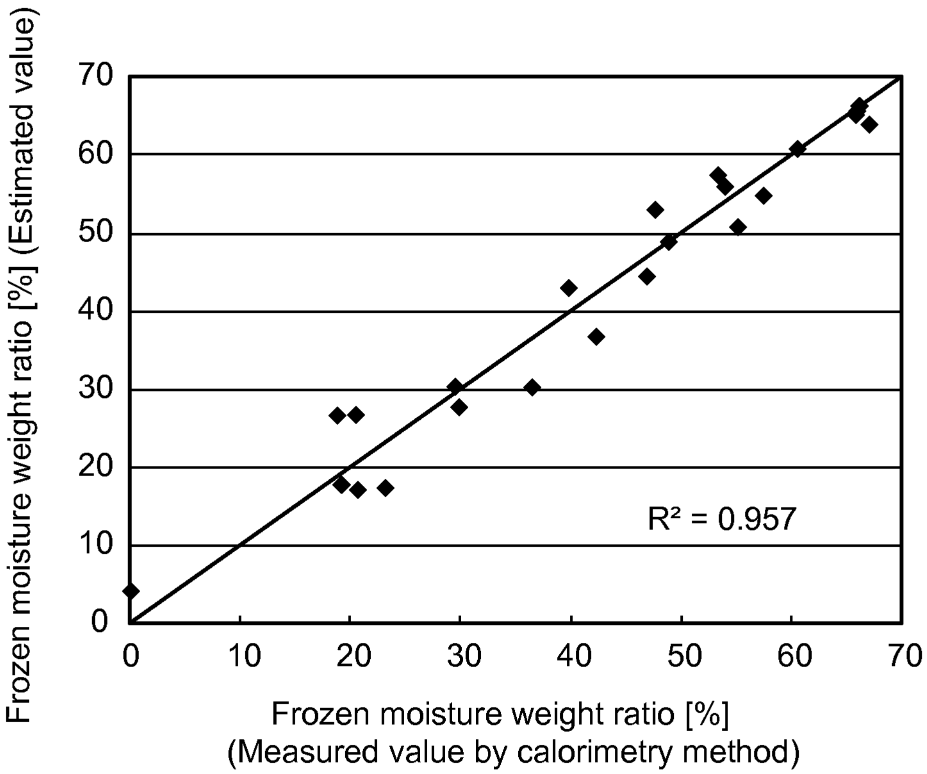
Frozen moisture weight ratio [%] (Estimated value)
70
60
50
40
30
20
10
0
0
10
20
30
40
50
60
70
R² = 0.957
Frozen moisture weight ratio [%]
(Measured value by calorimetry method)

FIG. 12A

[Surimi]

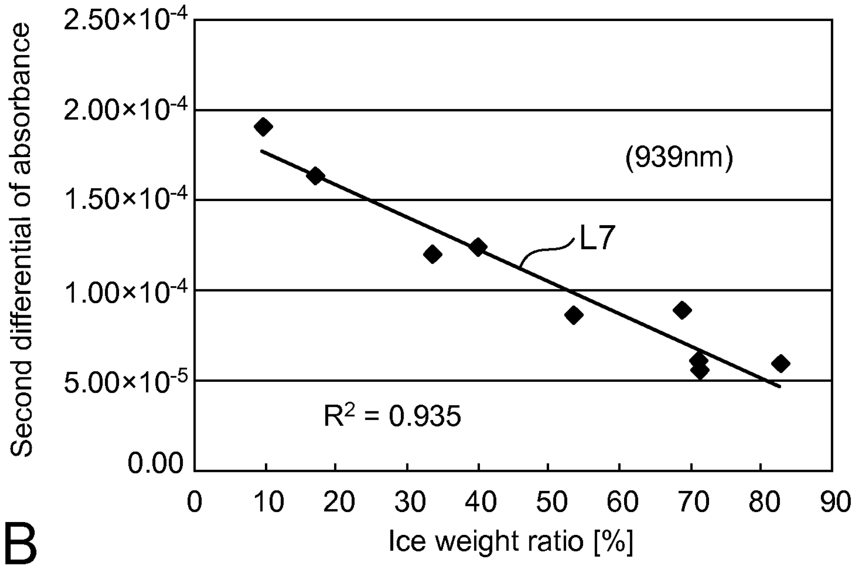


FIG. 12B

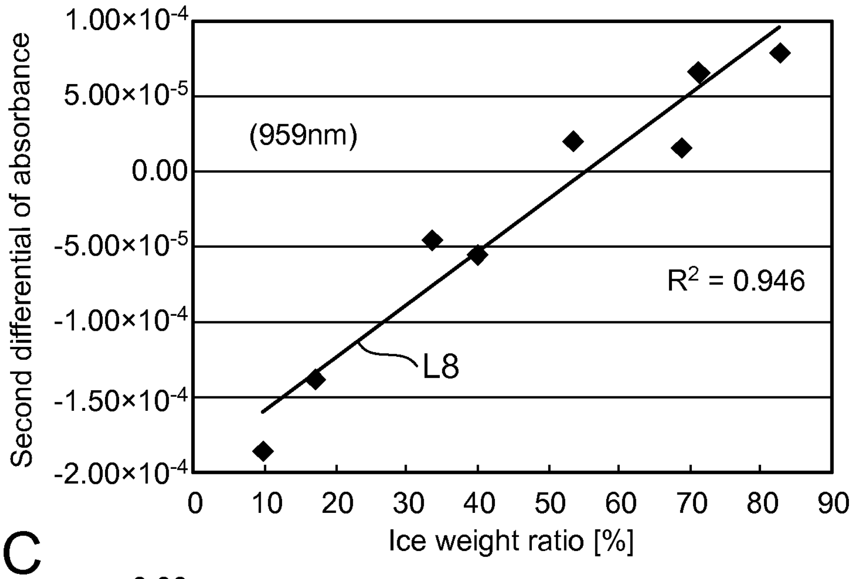


FIG. 12C

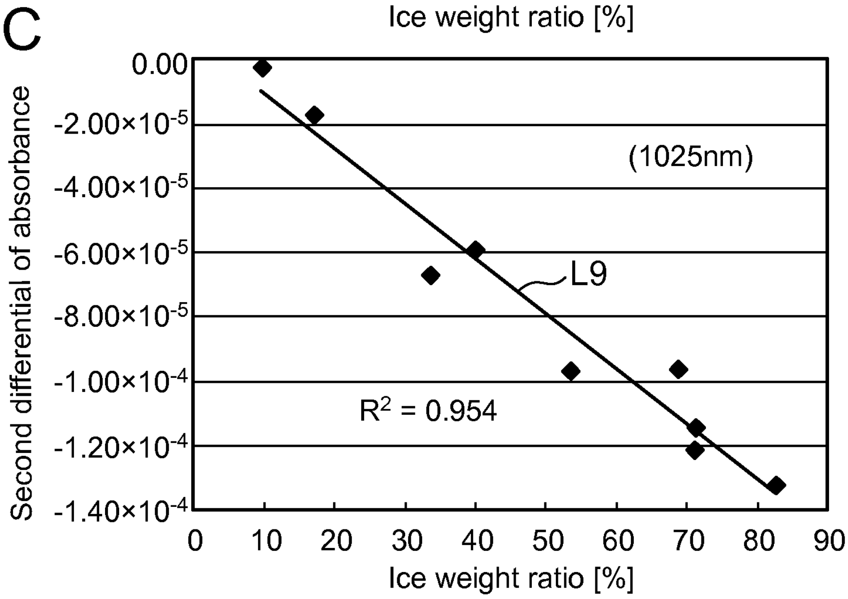

FIG. 13

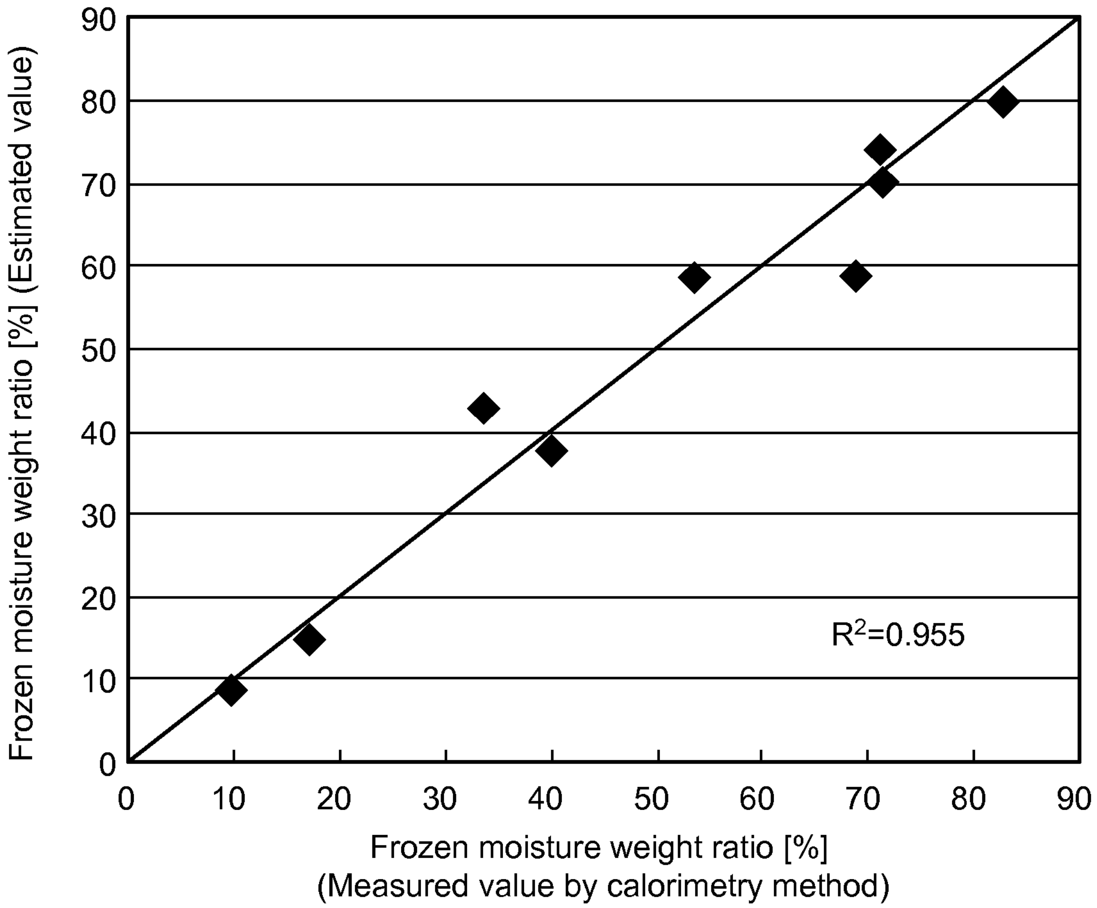
Frozen moisture weight ratio [%] (Estimated value)
90
80
70
60
50
40
30
20
10
0
R²=0.955
0
10
20
30
40
50
60
70
80
90
Frozen moisture weight ratio [%]
(Measured value by calorimetry method)

FIG. 14A

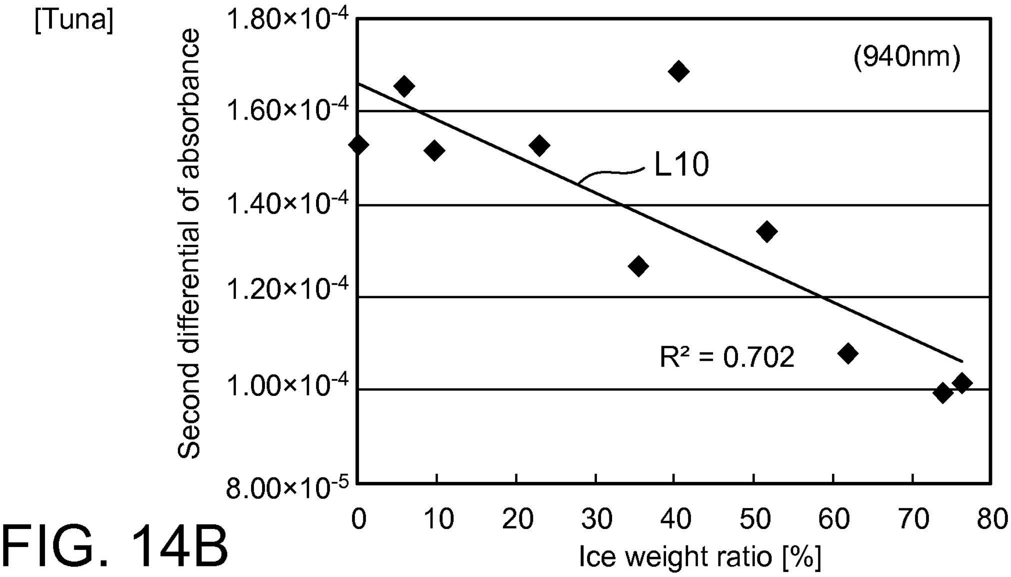


FIG. 14B

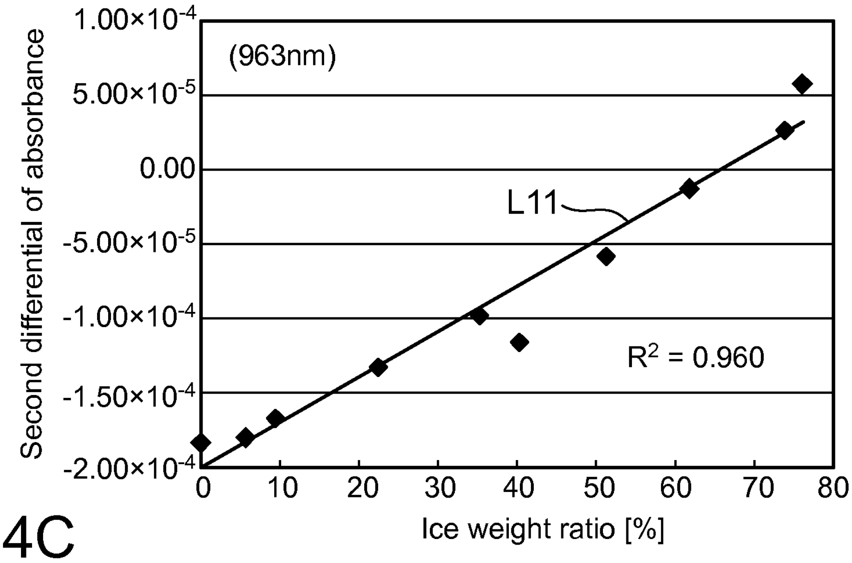


FIG. 14C

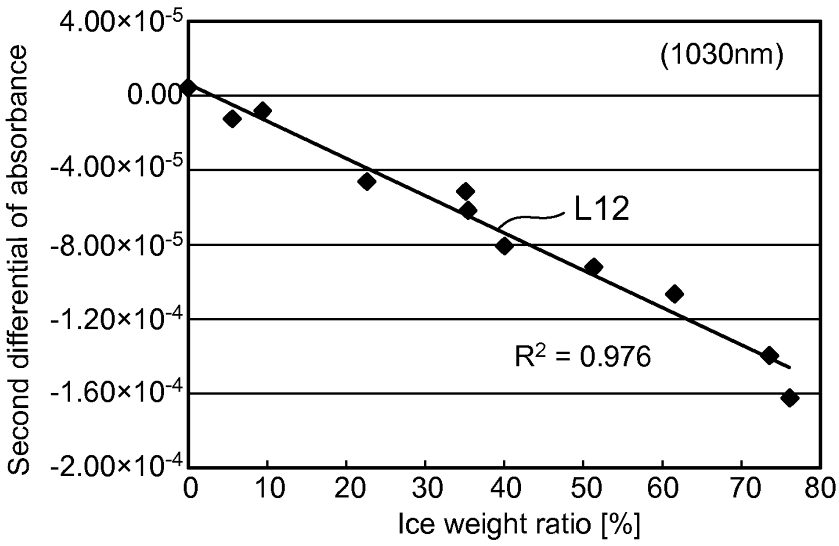

FIG. 15

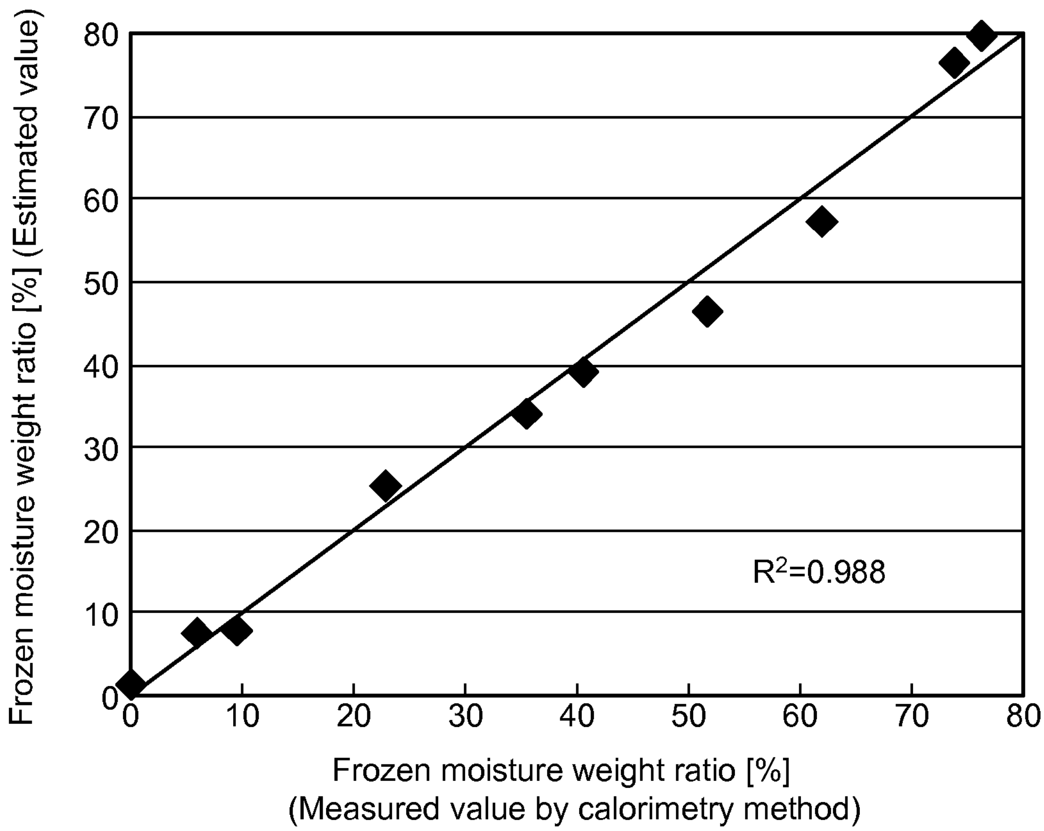
Frozen moisture weight ratio [%] (Estimated value)
80
70
60
50
40
30
20
10
0
0
10
20
30
40
50
60
70
80
R²=0.988
Frozen moisture weight ratio [%]
(Measured value by calorimetry method)

FIG. 16A

[Ham of pig]

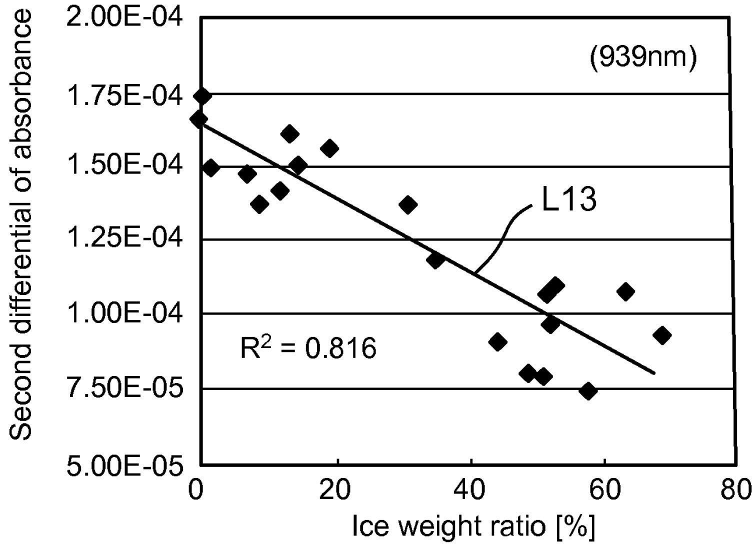


FIG. 16B

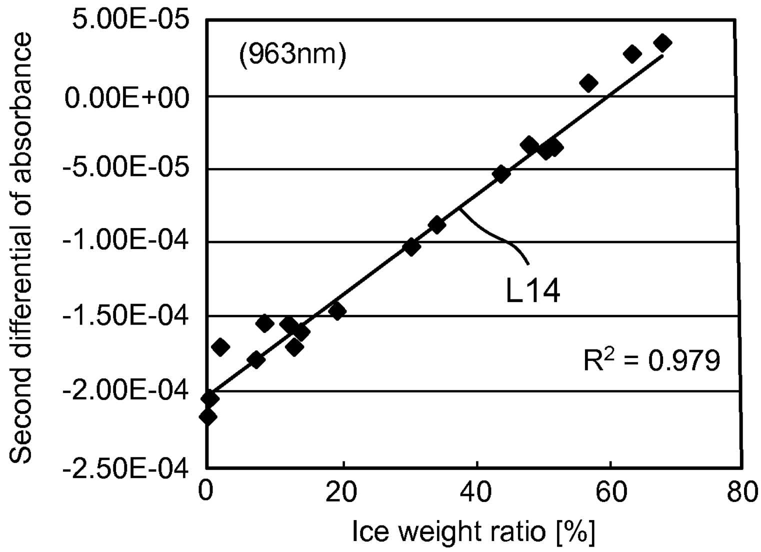


FIG. 16C

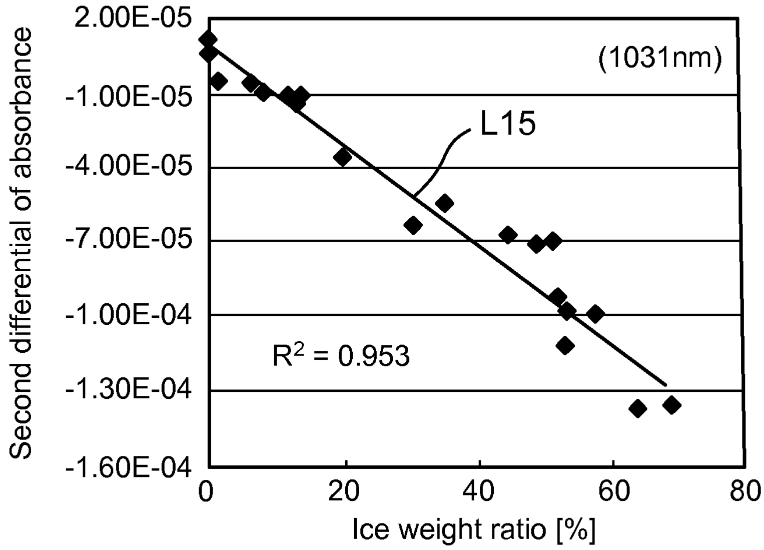

FIG. 17

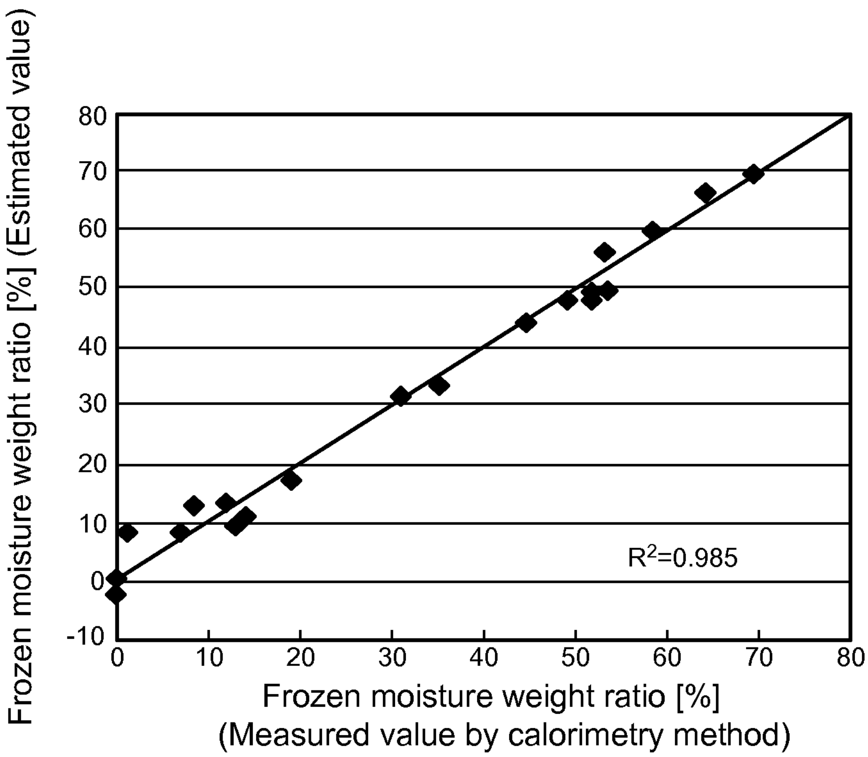
Frozen moisture weight ratio [%] (Estimated value)
80
70
60
50
40
30
20
10
0
-10
0
10
20
30
40
50
60
70
80
R2=0.985
Frozen moisture weight ratio [%]
(Measured value by calorimetry method)

FIG. 18A

[Agar]

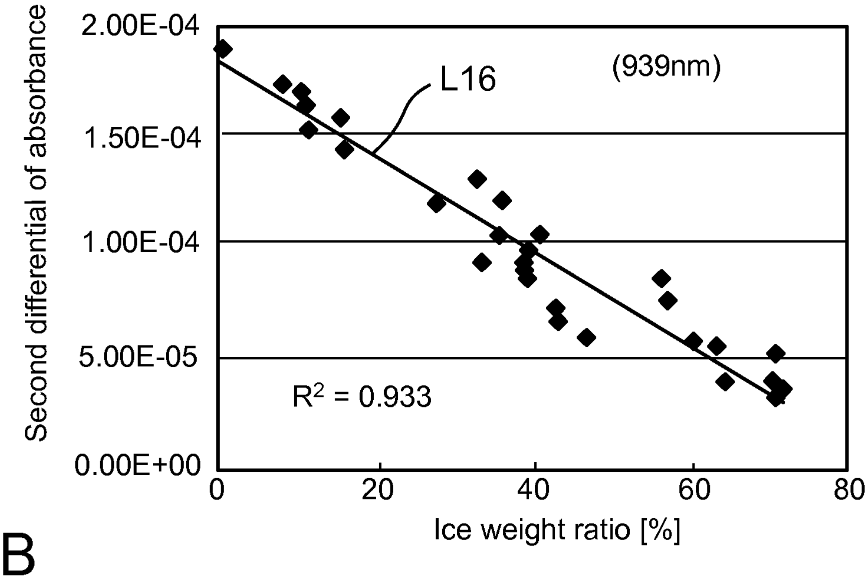


FIG. 18B

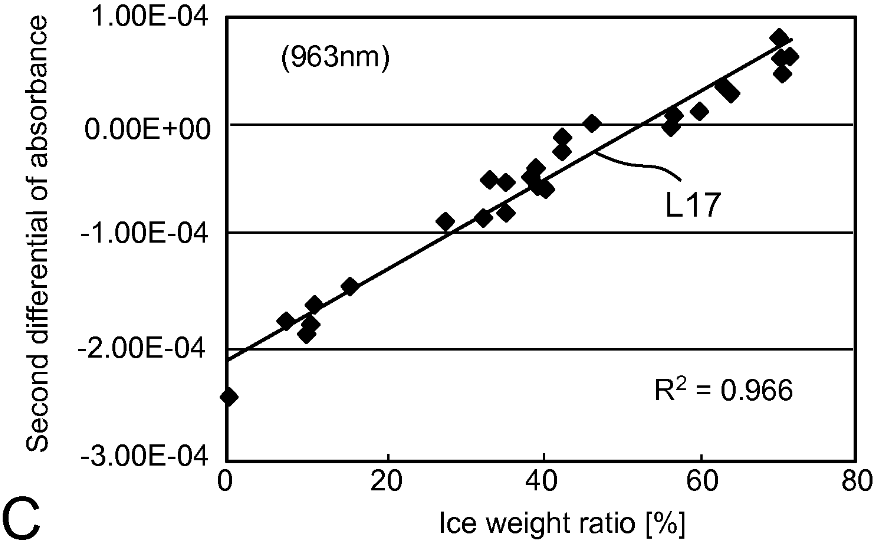


FIG. 18C

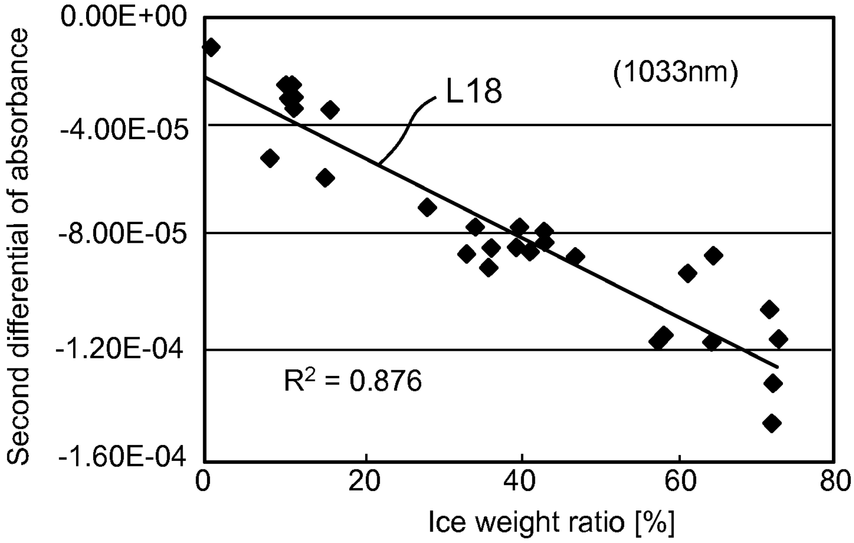

FIG. 19

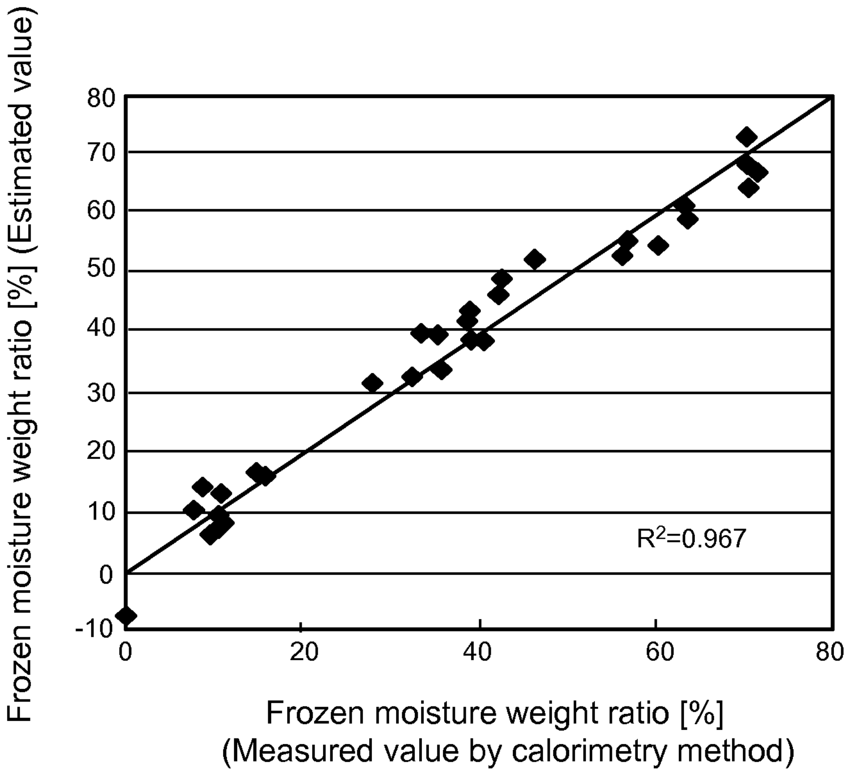
Frozen moisture weight ratio [%] (Estimated value)
80
70
60
50
40
30
20
10
0
-10
0
20
40
60
80
$R^2$=0.967
Frozen moisture weight ratio [%]
(Measured value by calorimetry method)

## INTERNATIONAL SEARCH REPORT

International application No.
PCT/JP2016/056558

A. CLASSIFICATION OF SUBJECT MATTER
*G01N21/359*(2014.01)i, *G01N21/3554*(2014.01)i, *G01N33/02*(2006.01)i

According to International Patent Classification (IPC) or to both national classification and IPC

B. FIELDS SEARCHED

Minimum documentation searched (classification system followed by classification symbols)
G01N21/00-21/61, G01N33/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| Y | OTTESTAD S, et.al., Prediction of ice fraction and fat content in super-chilled salmon by non-contact interactance near infrared imaging, Journal of Near Infrared Spectroscopy, 2009.03.23, Vol.17, No.2, p.77-87 | 1-8 |
| Y | Kabusu TO, "Shokuhin no Keisoku Gijutsu, Measuring method for three-dimensional structure of ice crystals in frozen materials by near-infrared imaging spectroscopy", The Food Industry, 30 January 2008 (30.01.2008), vol.51, no.4, pages 20 to 26 | 1-8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 May 2016 (16.05.16) | 31 May 2016 (31.05.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No. PCT/JP2016/056558

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2008-089529 A (Incorporated Administrative Agency Fisheries Research Agency), 17 April 2008 (17.04.2008), paragraphs [0008] to [0015]; fig. 1 to 3 (Family: none) | 1-8 |
| Y | JP 2010-074099 A (Sumitomo Electric Industries, Ltd.), 02 April 2010 (02.04.2010), paragraphs [0066] to [0069]; fig. 19, 20 & US 2011/0168895 A1 paragraphs [0134] to [0137]; fig. 19, 20 & WO 2010/032553 A1 & EP 2330630 A1 | 1-8 |
| Y | JP 9-119894 A (Norin Suisansho Chugoku Nogyo Shikenjo-cho), 06 May 1997 (06.05.1997), paragraphs [0004] to [0018]; fig. 1 to 7 (Family: none) | 1-8 |
| A | JP 8-159962 A (Tohoku Electric Power Co., Inc.), 21 June 1996 (21.06.1996), entire text; all drawings (Family: none) | 1-8 |
| A | US 2007/0188372 A1 (AGRESEARCH LTD.), 16 August 2007 (16.08.2007), entire text; all drawings & WO 2006/016824 A1 & EP 1776580 A & NZ 534673 A & CA 2576996 A | 1-8 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2014189501 A
- JP H0560685 A